# EUROPEAN PATENT APPLICATION

(11) **EP 4 520 316 A1**
(43) Date of publication of application: **12.03.2025**
(21) Application number: 23799438.9
(22) Date of filing: 17.04.2023
(51) Int. Cl.: A61K 8/894, A61Q 1/00, A61Q 1/04, A61Q 15/00, A61Q 17/04, A61Q 19/00

(54) **ACTIVATOR COMPOSITION AND COSMETIC**

(30) Priority: 02.05.2022 JP 2022076074
(71) Applicant: SHIN-ETSU CHEMICAL CO., LTD., Tokyo 1000005 (JP)
(72) Inventor: KONISHI Masayuki, Tokyo 100-0005 (JP)
(74) Representative: Mewburn Ellis LLP
(86) International application number: PCT/JP2023/015346
(87) International publication number: WO 2023/214506

(57) **Abstract**

Provided are: a surfactant composition containing
(A) a nonionic surfactant that has two or more hydroxyl groups per hydrophilic group, and
(B) an oily component that is in a liquid form at 25°C and has only one hydroxyl group,
the viscosity at 25°C of said surfactant composition being lower than 50% of the viscosity of component (A) and said surfactant composition having low viscosity and excellent handling properties; and a cosmetic containing the surfactant composition and giving good feeling in use.

## Description

### TECHNICAL FIELD

This invention relates to a surfactant composition and a cosmetic composition containing the same.

### BACKGROUND ART

In general, nonionic surfactants used in cosmetics are surfactants having in the molecule hydrophilic groups which are not dissociated into ions. Most often, polyoxyethylene chains and polyhydric alcohol chains are used as the hydrophilic group.

Nonionic surfactants having a polyhydric alcohol chain as represented by glycerin are widely used as an emulsifier, solubilizing agent or the like for their safety and functions. For example, Patent Document 1 discloses a milky W/O emulsion cosmetic having a low viscosity, a pleasant feeling on use, and satisfactory age stability. This emulsion cosmetic is characterized by comprising one or more polyglycerin fatty acid esters, glycerin, oil base, and water.

It is known from Patent Document 2 that glycerin or polyglycerin-modified silicone base surfactants have excellent moisturizing performance. It is known from Patent Document 3 to increase the dispersibility of hydrophobized powder for improving the stability of formulations. It is also known from Patent Document 4 to combine a glycerin-modified silicone base surfactant with trimethylsiloxysilicic acid for improving the stability of formulations.

However, the nonionic surfactants based on a polyhydric alcohol chain having two or more hydroxy groups as hydrophilic groups are difficult to handle because of viscosity buildup. In particular, the development of high-molecular-weight compounds therefrom is limited.

### PRIOR ART DOCUMENTS

### PATENT DOCUMENTS

Patent Document 1: JP-A H06-128135
Patent Document 2: JP-A 2002-003334
Patent Document 3: JP-A 2013-035872
Patent Document 4: JP-A 2007-182402

### SUMMARY OF INVENTION

### TECHNICAL PROBLEM

An object of the invention, which has been made under the above-mentioned circumstances, is to provide a surfactant composition having a low viscosity and ease of handling, and a cosmetic composition comprising the same and giving a pleasant feeling on use.

### SOLUTION TO PROBLEM

Making extensive investigations to attain the above object, the inventor has found that the outstanding problem can be overcome by providing a surfactant composition comprising (A) a nonionic surfactant having hydrophilic groups and at least two hydroxy groups per hydrophilic group and (B) an oily component having only one hydroxy group, and controlling the viscosity at 25°C of the surfactant composition to less than 50% of the viscosity of component (A). The invention is predicated on this finding.

Accordingly, the invention provides a surfactant composition and a cosmetic, as defined below.
1. A surfactant composition comprising
   (A) a nonionic surfactant having hydrophilic groups and at least two hydroxy groups per hydrophilic group, and
   (B) an oily component having only one hydroxy group, which is liquid at 25°C, the surfactant composition having a viscosity at 25°C which is less than 50% of the viscosity of component (A).
2. The surfactant composition of 1 wherein the ratio of component (A) to component (B) is from 70:30 to 95:5 in weight ratio, the surfactant composition having a viscosity at 25°C of 100 to 50,000 mPa·s.
3. The surfactant composition of 1 or 2 wherein component (A) is a polyglycerin-modified silicone.
4. The surfactant composition of 3 wherein component (A) is a straight or branched polyglycerin-modified silicone.
5. The surfactant composition of any one of 1 to 4 wherein component (B) is at least one compound selected from aliphatic alcohols and ester oils.
6. The surfactant composition of 5 wherein component (B) is a branched aliphatic alcohol of 16 to 24 carbon atoms.
7. The surfactant composition of any one of 1 to 6 wherein component (B) has a solubility of less than 1 g in 100 g of purified water at 25°C.
8. A cosmetic composition comprising 0.1 to 40% by weight, based on the overall cosmetic composition, of the surfactant composition of any one of 1 to 7.
9. The cosmetic composition of 8 which is a water-in-oil emulsion cosmetic composition.

### ADVANTAGEOUS EFFECTS OF INVENTION

According to the invention, there are provided a surfactant composition which is improved in handling by lowering the viscosity of a nonionic surfactant having at least two hydroxy groups per hydrophilic group, and a cosmetic composition comprising the same and giving a pleasant feeling on use.

### DESCRIPTION OF EMBODIMENTS

Now the invention is described in detail. Herein, the name of ingredients is sometimes expressed by the Japanese labeling name or the International Nomenclature for Cosmetic Ingredients (INCI). When the Japanese labeling name is identical with the INCI, sometimes either one is omitted.

### [Component (A)]

Component (A) is a nonionic surfactant having hydrophilic groups and at least two hydroxy groups per hydrophilic group, which may be used alone or in admixture of two or more. The hydrophilic groups in component (A) are not particularly limited as long as they are commonly used in nonionic surfactants. Examples include polyhydric alcohol structure-bearing compounds such as glycerin, trimethylolpropane, pentaerythritol, sorbitan, sorbitol, sucrose, polyglycerin, and raffinose saccharide. The foregoing hydrophilic groups in which some hydroxy groups thereof have been modified are included as well as those structures obtained by combining hydrophilic groups of single type or two or more different types. Among others, hydrophilic groups having glycerin or polyglycerin structure are preferred from the standpoints of emulsification, dispersion and moisture retention.

The number of hydroxy groups in component (A) is at least 2 per hydrophilic group. Although the benefits of the invention become more outstanding with a greater number of hydroxy groups, the number of hydroxy groups is preferably 3 to 20, more preferably 3 to 15, even more preferably 4 to 10 in view of a balance with hydrophobic groups.

Examples of component (A) include surfactants based on fatty acid esters of polyhydric alcohols such as sorbitan fatty acid esters, polyoxyethylene sorbitan fatty acid esters, polyoxyethylene sorbitol fatty acid esters, glycerin fatty acid esters, polyoxyethylene glycerin fatty acid esters, and polyglycerin fatty acid esters, and silicone-based surfactants such as polyglycerin-modified silicone (or organopolysiloxane). Of these, silicone-based surfactants are preferred because the benefits of the invention become more outstanding, with polyglycerin-modified silicone being more preferred.

Examples of the ester-based surfactant include polyglyceryl-2 isostearate, polyglyceryl-10 laurate, polyhydroxystearic acid, sorbitan stearate, and sorbitan sesquiisostearate.

The silicone-based surfactants may be either straight or branched. Included are partially crosslinked polyglycerin-modified silicones, straight or branched polyglycerin-modified organopolysiloxanes, and straight or branched polyglycerin/alkyl-co-modified organopolysiloxanes. Inter alia, those based on straight or branched organopolysiloxane as main skeleton and having polyglycerin chains as side chains and those based on straight or branched organopolysiloxane as main skeleton and having polyglycerin and alkyl chains as side chains are most preferably used from the aspects of feeling on use and emulsifying capability.

Examples of the partially crosslinked polyglycerin-modified silicone include (dimethicone/polyglycerin-3) crosspolymer (INCI), (lauryl dimethicone/polyglycerin-3) crosspolymer (INCI), and (polyglycerin-3/lauryl polydimethylsiloxyethyl dimethicone) crosspolymer (INCI). The partially crosslinked polyglycerin-modified silicone may also be a composition consisting of the partially crosslinked polyglycerin-modified silicone and an oil which is liquid at room temperature. Examples of the liquid oil include cyclopentasiloxane (INCI), dimethicone (INCI), mineral oil (INCI), isododecane (INCI), triethylhexanoin (INCI), and squalane (INCI).

Commercially available examples of the partially crosslinked polyglycerin-modified silicone which is swollen with a liquid oil include KSG-710, KSG-810, KSG-820, KSG-830, KSG-840, KSG-820Z, and KSG-850Z by Shin-Etsu Chemical Co., Ltd.

Examples of the straight or branched polyglycerin-modified organopolysiloxane and straight or branched polyglycerin/alkyl-co-modified organopolysiloxane include polyglyceryl-3 disiloxane dimethicone (INCI), polyglyceryl-3 polydimethylsiloxyethyl dimethicone (INCI), lauryl polyglyceryl-3 polydimethylsiloxyethyl dimethicone (INCI), and bis-butyldimethicone polyglyceryl-3 (INCI). Commercially available examples include KF-6100, KF-6104, KF-6105, KF-6106, and KF-6115 by Shin-Etsu Chemical Co., Ltd. Since the partially crosslinked polyglycerin-modified silicone has a viscosity-increasing effect owing to the crosslinked section in its structure, the viscosity-reducing effect of component (B) combined therewith is not outstanding. The polyglycerin-modified silicone is preferably straight or branched.

The viscosity at 25°C of component (A) is preferably selected, for example, in the range of 100 to 500,000 mPa·s, more preferably 1,000 to 400,000 mPa·s, even more preferably 3,000 to 500,000 mPa·s. Even when component (A) has a viscosity in the range, the invention is successful in reducing viscosity. As used herein, the viscosity is measured at 25°C by the method using a Brookfield rotational viscometer described in JIS K7117-1: 1999 (the same holds true, hereinafter).

### [Component (B)]

Component (B) is an oily component having only one hydroxy group, which is liquid at 25°C. The preferred component (B) has a solubility of less than 1 g in 100 g of purified water at 25°C. Regardless of the number of hydroxy groups in component (A), oils having no hydroxy group and oils having two or more hydroxy groups are excluded from component (B). Lower alcohols of 1 to 5 carbon atoms which are so highly hydrophilic as to adversely affect affinity to an oil layer are also excluded from component (B).

Examples of component (B) include higher alcohols, higher fatty acids, and ester oils which are liquid at 25°C. Inter alia, higher alcohols such as aliphatic alcohols and ester oils are preferred. Branched chain aliphatic alcohols of 16 to 24 carbon atoms are most preferred in view of a feeling on use and viscosity lowering. These oils may be used alone or in admixture of two or more.

Suitable higher alcohols include alcohols of 6 or more carbon atoms, preferably 16 to 24 carbon atoms, and more preferably 16 to 20 carbon atoms. Alcohols of 16 to 18 carbon atoms are most preferred in view of compatibility with component (A) and a feeling on use. Examples of the higher alcohol include lauryl alcohol (INCI), hexyl decanol (INCI), oleyl alcohol (INCI), isostearyl alcohol (INCI), octyl dodecanol (INCI), and decyl tetradecanol (INCI).

Suitable higher fatty acids include fatty acids of 6 or more carbon atoms, preferably 16 to 24 carbon atoms, and more preferably 16 to 20 carbon atoms. Examples include oleic acid (labeling name, INCI: Oleic Acid), linoleic acid (labeling name, INCI: Linoleic Acid), linolenic acid (labeling name, INCI: Linolenic Acid), arachidonic acid (labeling name, INCI: Arachidonic Acid), eicosapentaenoic acid (labeling name, INCI: Eicosapentaenoic Acid), docosahexaenoic acid (labeling name, INCI: Docosahexaenoic Acid), and isostearic acid (labeling name, INCI: Isostearic Acid).

Examples of the ester oil include diisostrearyl malate (labeling name, INCI: Diisostearyl Malate), triethyl citrate (labeling name, INCI: Triethyl Citrate), cetyl lactate (labeling name, INCI: Cetyl Lactate), myristyl lactate (labeling name, INCI: Myristyl Lactate), cholesteryl hydroxystearate (labeling name, INCI: Cholesteryl Hydroxystearate), coconut oil fatty acid BG (labeling name, INCI: Butylene Glycol Cocoate), polyglyceryl-2 triisostearate (labeling name, INCI: Polyglyceryl-2 Triisostearate), Homosalate (INCI), and ethylhexyl salicylate (labeling name, INCI: Ethylhexyl Salicylate).

The invention intends to reduce the viscosity of component (A) by combining component (A) with component (B). The viscosity at 25°C of component (B) is not limited as long as it is liquid at 25°C. The viscosity is preferably selected, for example, in the range of 1 to 3,000 mPa·s, more preferably 5 to 400 mPa·s, even more preferably 30 to 60 mPa·s.

The ratio of component (A) to component (B) is preferably from 70:30 to 95:5, more preferably from 80:20 to 98:2, even more preferably from 85:15 to 95:5, as expressed in weight ratio. Provided that the total of components (A) and (B) is 100, if the weight ratio of component (A) exceeds 95, a fully low viscosity may not be achieved; and if the weight ratio of component (B) exceeds 30, a sufficient surfactant function may not be obtained. Although it suffices that the total content of components (A) and (B) be at least 15% by weight of the surfactant composition, the total content is preferably at least 90% by weight, more preferably at least 95% by weight, even more preferably at least 99% by weight, most preferably 100% by weight. When the total content of components (A) and (B) is at least 90% by weight, the ease of mixing with an oil phase (or ease of metering) during preparation of a cosmetic composition is improved. Since the surfactant composition of the invention is prepared by previously mixing components (A) and (B) and then blended in a cosmetic formulation, the surfactant composition having a low viscosity and ease of handling is easy to meter and admit. In this sense, the surfactant composition is not a mixture which is obtained from unintentional or incidental mixing of components during formulation.

In addition to components (A) and (B), the surfactant composition contains other components which are commonly blended in cosmetics. The type and amount of other components are not particularly limited as long as a viscosity in the predetermined range is obtained. Suitable other components include surfactants other than component (A) such as polyether-modified silicones, pyrrolidone carboxylic acid-modified silicones, and ionic surfactants, oils other than component (B) such as silicone oils, hydrocarbon oils and ester oils, aqueous components, and powders.

The mixture of components (A) and (B) and the surfactant composition should have a viscosity at 25°C which is less than 50%, preferably less than 40%, more preferably less than 30%, even more preferably less than 25% of the viscosity of component (A). The mixture and the surfactant composition should preferably have a viscosity at 25°C of 100 to 50,000 mPa·s, more preferably 500 to 15,000 mPa·s, even more preferably 500 to 5,000 mPa·s, because the viscosity of component (A) can be further reduced. The composition having a viscosity in the range is easy to handle and easy to meter or mix. As mentioned above, the viscosity is measured at 25°C by the method using a B-type rotational viscometer described in JIS K7117-1: 1999.

The surfactant composition is preferably a composition which can be uniformly mixed. Specifically, it is a composition which does not separate into components under 25°C conditions after mixing. A composition which turns white turbid is unfavorable. A composition which is dissolved semitransparent is preferred, and a composition which is dissolved transparent is more preferred from the aspect of quality control. With respect to the evaluation of transparency, when a cell of 1 cm thick is filled with the composition and measured for total light transmission, the composition is judged semitransparent for a transmission of at least 50% and transparent for a transmission of at least 80%. Preferred from the aspect of transparency are a combination of polyglyceryl-3 polydimethylsiloxyethyl dimethicone as component (A) with a higher alcohol of 16 to 18 carbon atoms as component (B), a combination of lauryl polyglyceryl-3 polydimethylsiloxyethyl dimethicone as component (A) with a higher alcohol of 16 to 24 carbon atoms as component (B), a combination of polyglyceryl-3 disiloxane dimethicone as component (A) with a higher alcohol of 16 to 18 carbon atoms as component (B), and a combination of polyglyceryl-10 laurate as component (A) with a higher alcohol of 16 to 18 carbon atoms as component (B).

The surfactant composition is obtained by mixing components (A) and (B) and optional components. The optional components include the below-exemplified ingredients used in cosmetics and may be blended in suitable amounts.

### [Cosmetic composition]

The surfactant composition may be used in a variety of applications, especially as a raw material for all cosmetic compositions for external use on the skin and hair. A cosmetic composition having the surfactant composition blended therein gives a pleasant feeling on use. The cosmetic composition of the invention is obtained by blending the previously prepared surfactant composition in a cosmetic formulation. Herein, the amount of the surfactant composition blended is preferably in a range of 0.1 to 40% by weight of the overall cosmetic composition. Particularly when the surfactant composition is used as an emulsifier during preparation of an emulsion, the amount is more preferably 0.1 to 8% by weight, even more preferably 0.1 to 5% by weight. The total amount of components (A) and (B) is preferably 0.1 to 8% by weight, more preferably 0.1 to 5% by weight of the overall cosmetic composition.

The cosmetic composition comprising the surfactant composition of the invention may take any of forms, for example, powder, oily liquid, water-in-oil (W/O) emulsion cosmetics, oil-in-water (O/W) emulsion cosmetics, nonaqueous cosmetics, multiple emulsions of W/O/W or O/W/O type. Particularly when the cosmetic composition is used as a surfactant for W/O emulsion cosmetics, a stable cosmetic composition is obtained because a lowering of viscosity brings on an improvement in emulsification efficiency.

In the cosmetic composition of the invention, various optional components commonly used in conventional cosmetics can be blended.

### <Other optional components>

As the other optional component, the cosmetic composition may contain, for example, (1) oils other than component (B), (2) aqueous components, (3) surfactants other than component (A), (4) powders, (5) compositions consisting of crosslinked organopolysiloxane and an oil which is liquid at room temperature, (6) film-forming agents, (7) UV-absorbing/scattering agents, and (8) other additives, which may be used alone or in admixture of two or more and in suitable amounts.

### (1) Oils other than component (B)

In the inventive cosmetic composition, oils other than component (B) may be blended. The oils may be volatile or non-volatile and solid, semisolid or liquid at room temperature (25°C). For example, silicone oil, silicone wax, naturally occurring animal and plant oils and fats and semi-synthetic oils and fats, hydrocarbon oils, higher alcohols, fatty acids, ester oils, fluorinated oils, and UV absorbers.

### • Silicone oil

Examples of the silicone oil include dimethicone (INCI), trisiloxane (INCI), alkyl-modified silicones such as methyl trimethicone (INCI), ethyl trisiloxane (INCI), ethyl methicone (INCI), and hexyl dimethicone (INCI), long chain alkyl-modified silicones such as caprylyl methicone (INCI), low to high viscosity straight or branched organopolysiloxanes such as phenyl trimethicone (INCI), diphenyl dimethicone (INCI), diphenylsiloxyphenyl trimethicone (INCI), tetraphenyl dimethyldisiloxane (INCI), and methylhydrogenpolysiloxane, cyclic organopolysiloxanes such as cyclotetrasiloxane (INCI), cyclopentasiloxane (INCI), and cyclohexasiloxane (INCI), amino-modified organopolysiloxanes such as amodimethicone (INCI) and aminopropyl dimethicone (INCI), pyrrolidone-modified organopolysiloxanes such as PCA dimethicone (INCI), pyrrolidone carboxylic acid-modified organopolysiloxanes, silicone rubbers such as gum-like dimethylpolysiloxane having a high degree of polymerization, gum-like amino-modified organopolysiloxane, and gum-like dimethylsiloxane-methylphenylsiloxane copolymers, as well as solutions of low viscosity organopolysiloxanes such as silicone gum and rubber, amino acid-modified silicones, fluorine-modified silicones, silicone resins, and silicone resin solutions.

Commercially available examples of the silicone oil include KF-96L-1cs, KF-96L-1.5cs, KF-96L-2cs, KF-96L-6cs, KF-4422, KF-54, KF-54HV, KF-56A, and KF-995 by Shin-Etsu Chemical Co., Ltd.

### • Solid oily component

When it is desired that the cosmetic composition be solidified, an oily component which is solid at 25°C is preferably blended. Examples of the oily component which is solid at 25°C include waxes, hydrocarbons, esters, higher alcohols, and higher fatty acids having a melting point of preferably at least 40°C, more preferably 60 to 110°C. The oily component is not particularly limited as long as it is commonly blended in cosmetics. Specific examples include plant waxes such as carnauba wax (INCI: Copernicia Cefifera (Carnauba) Wax), sugar cane wax, candelilla wax (INCI: Euphorbia Cerifera (Candelilla) Wax), purified candelilla wax, rice wax, Japan wax, jojoba wax, capok wax, rice bran wax, Myrica Cerifera Fruit Wax, shea butter, cacao butter, Japan wax (INCI: Rhus Succedanea Fruit Wax), montan wax (INCI: Montan Wax), and hydrogenated castor oil isostearate; animal waxes such as beeswax, head, beef bone oil, lard (INCI: Lard), horse fat (INCI: Horse Fat), tallow, lanolin (INCI: Lanolin), insect wax, shellac wax and sperm wax; semi-synthetic waxes such as lanolin ester, lanolin fatty acid esters, beeswax acid esters; hydrogenated oils such as hydrogenated castor oil and hydrogenated coconut oil; hydrocarbon waxes such as solid paraffin, polyethylene, ceresin, ozokerite, and microcrystalline wax; wax esters such as synthetic beeswax; amic acid stearyl alcohols such as dioctyldodecyl lauroylglutaminate, dioctyldodecyl lauroylglutaminate, and dioctyldodecyl lauroylglutaminate; fatty acids such as stearic acid and behenic acid; and silicone waxes such as acrylate silicone resins in the form of acrylate silicone graft or block copolymers (acrylate silicone graft copolymers: KP-561P, 562P by Shin-Etsu Chemical Co., Ltd.) or derivatives thereof. Any one or more compounds selected from the foregoing is preferred.

### • Natural animal and plant oils and fats and semi-synthetic oils and fats

Examples of the natural animal and plant oils and fats and semi-synthetic oils and fats include naturally occurring plant oils such as avocado oil (labeling name, INCI: Persea Gratissima (Avocado) Oil), linseed oil (labeling name, INCI: Linum Usitatissimum (Linseed) Seed Oil), almond oil (labeling name, INCI: Prunus Amygdalus Dulcis (Sweet Almond) Oil), perilla oil (labeling name), olive oil (labeling name, INCI: Olea Europaea (Olive) Fruit Oil), Torreya California oil (labeling name, INCI: Torreya Californica (Californica Nutmeg) Oil), citronella grass oil (labeling name, INCI: Cymbopogon Nardus (Citronella) Oil), kaya seed oil (labeling name, INCI: Torreya Nucifera Seed Oil), kyounin oil (labeling name, INCI: Kyounin Yu), wheat germ oil (labeling name, INCI: Triticum Vulgare (Wheat) Germ Oil), sesame oil (labeling name, INCI: Sesamum Indicum (Sesame) Seed Oil), wheat germ oil (labeling name, INCI: Triticum Vulgare (Wheat) Germ Oil); germ oils such as rice germ oil (labeling name, INCI: Oryza Sativa (Rice) Germ Oil), rice bran oil (labeling name, INCI: Oryza Sativa (Rice) Bran Oil), camellia oil (labeling name, INCI: Camellia Kissi Seed Oil), safflower oil (labeling name, INCI: Carthamus Tinctorius (Safflower) Seed Oil), soybean oil (labeling name, INCI: Glycine Soja (Soybean) Oil), gold tea oil (labeling name, INCI: Camellia Sinensis Seed Oil), tea seed oil (labeling name, INCI: Camellia Japonica Seed Oil), evening primrose oil (labeling name, INCI: Oenothera Biennis (Evening Primrose) Oil), rapeseed oil (labeling name), corn germ oil (labeling name, INCI: Zea Mays (Corn) Germ Oil), wheat germ oil (labeling name, INCI: Triticum Vulgare (Wheat) Germ Oil); natural plant oils such as persic oil (labeling name), palm oil (labeling name, INCI: Elaeis Guineensis (Palm) Oil), palm kernel oil (labeling name, INCI: Elaeis Guineensis (Palm) Kernel Oil), castor oil (labeling name, INCI: Ricinus Communis (Castor) Seed Oil), sunflower oil (labeling name, INCI: Helianthus Annuus (Sunflower) Seed Oil), grape seed oil (labeling name, INCI: Vitis Vinifera (Grape) Seed Oil), jojoba seed oil (labeling name, INCI: Simmondsia Chinensis (Jojoba) Seed Oil), macadamia seed oil (labeling name, INCI: Macadamia Ternifolia Seed Oil), meadowfoam oil (labeling name, INCI: Limnanthes Alba (Meadowfoam) Seed Oil), cotton seed oil (labeling name, INCI: Gossypium Herbaceum (Cotton) Seed Oil), coconut oil (labeling name, INCI: Cocos Nucifera (Coconut) Oil), and peanut oil (labeling name, INCI: Archis Hypogaea (Peanut) Oil); natural animal oils such as shark liver oil (labeling name, INCI: Shark Liver Oil), cod liver oil (labeling name, INCI: Cod Liver Oil), fish liver oil (labeling name, INCI: Fish Liver Oil), turtle oil (labeling name, INCI: Turtle Oil), mink oil (labeling name, INCI: Mink Oil), and egg oil (labeling name, INCI: Egg Oil); and semi-synthetic oils and fats such as hydrogenated coconut oil (labeling name, INCI: Hydrogenated Coconut Oil) and liquid lanolin (labeling name, INCI: Lanolin Oil).

### • Hydrocarbon oil

The hydrocarbon oils include straight or branched hydrocarbon oils while they may be either volatile or nonvolatile.

Examples of the hydrocarbon oil include olefin oligomers (INCI), isoparaffins such as (C13, C14) isoparaffins (INCI), isododecane (INCI), undecane (INCI), dodecane (INCI), isohexadecane (INCI), hydrogenated polyisobutene (labeling name, INCI: Hydrogenated Polyisobutene), squalane (INCI), mineral oil (INCI), and alkanes such as coconut alkane (INCI), (C13-15) alkane (INCI), and Vaseline (labeling name, INCI Petrolatum).

### • Higher fatty acid

Exemplary of the higher fatty acid is hydroxystearic acid (labeling name, INCI: Hydroxystearic Acid).

### • Higher alcohol

Examples of the higher alcohol include straight saturated alcohols of 6 or more carbon atoms such as myristyl alcohol (INCI), cetyl alcohol (INCI), stearyl alcohol (INCI) and behenyl alcohol (INCI), as well as batyl alcohol (INCI). Also included are sterols such as cholesterol (INCI), sitosterol (labeling name, INCI: Beta-Sitosterol), phytosterol (INCI), and lanosterol (INCI).

### • Ester oil

Examples of the ester oil include diisobutyl adipate (labeling name, INCI: Diisobutyl Adipate), dihexyldecyl adipate (labeling name), diheptylundecyl adipate (labeling name, INCI: Diheptylundecyl Adipate), isostearyl isostearate (labeling name, INCI: Isostearyl Isostearate), isocetyl isostearate (labeling name, INCI: Isocetyl Isostearate), trimethylolpropane triisostearate (labeling name, INCI: Trimethylolpropane Triisostearate), glycol diethylhexanoate (labeling name, INCI: Glycol Diethylhexanoate), cetyl ethylhexanoate (labeling name, INCI: Cetyl Ethylhexanoate), trimethylolpropane triethylhexanoate (labeling name, INCI: Trimethylolpropane Triethylhexanoate), pentaerythrityl tetraethylhexanoate (labeling name, INCI: Pentaerythrityl Tetraethylhexanoate), cetyl octanoate (labeling name, INCI: Cetyl Ethylhexanoate), octyldodecyl esters such as octyldodecyl stearoyloxystearate (labeling name, INCI: Octyldodecyl Stearoyl Stearate), oleyl oleate (labeling name, INCI: Oleyl Oleate), octyldodecyl oleate (labeling name, INCI: Octyldodecyl Oleate), decyl oleate (labeling name, INCI: Decyl Oleate), neopentyl glycol dioctanoate (labeling name, INCI: Neopentyl Glycol Diethylhexanoate), neopentyl glycol dicaprate (labeling name, INCI: Neopentyl Glycol Dicaprate), diethylhexyl succinate (labeling name, INCI: Diethylhexyl Succinate), amyl acetate (labeling name, INCI: Amyl Acetate), ethyl acetate (labeling name, INCI: Ethyl Acetate), butyl acetate (labeling name, INCI: Butyl Acetate), isocetyl stearate (labeling name, INCI: Isocetyl Stearate), butyl stearate (labeling name, INCI: Butyl Stearate), diisopropyl sebacate (labeling name, INCI: Diisopropyl Sebacate), diethylhexyl sebacate (labeling name, INCI: Diethylhexyl Sebacate), isononyl isononanoate (labeling name, INCI: Isononyl Isononanoate), isotridecyl isononanoate (labeling name, INCI: Isotridecyl Isononanoate), palmitates such as isopropyl palmitate (labeling name, INCI: Isopropyl Palmitate), ethylhexyl palmitate (labeling name, INCI: Ethylhexyl Isopalmitate), and hexyldecyl palmitate (labeling name, INCI: Isocetyl Palmitate, Hexyldecyl Palmitate), myristates such as isopropyl myristate (labeling name, INCI: Isopropyl Myristate), octyldodecyl myristate (labeling name, INCI: Octyldodecyl Myristate), and myristyl myristate (labeling name, INCI: Myristyl Myristate), ethylhexyl laurate (labeling name, INCI: Ethylhexyl Laurate), hexyl laurate (labeling name, INCI: Hexyl Laurate), dioctyldodecyl lauroyl glutamate (labeling name, INCI: Dioctyldodecyl Lauroyl Glutamate), isopropyl lauroyl sarcosinate (labeling name, INCI: Isopropyl Lauroyl Sarcosinate), and coco-alkyl caprylate-caprate (labeling name, INCI: Coco-Caprylate-Caprate).

Glyceride oils are also included in the ester oils, such as triethylhexanoin (INCI), glyceryl tri(caprylate/caprate) (labeling name, INCI: Caprylic/Capric Triglyceride), and triglyceryl caprylate/caprate/succinate (labeling name, INCI: Caprylic/Capric/Succinic Triglyceride).

### • Fluorochemical oils

Examples of the fluorochemical oil include perfluorodecalin (INCI), perfluorononyl dimethicone (INCI), and perfluoromethylcyclopentane (INCI).

### • UV absorbers

Suitable UV absorbers include octocrylene (INCI), t-butyl methoxydibenzoylmethane (labeling name, INCI: Butyl Methoxydibenzoylmethane), diethylaminohydroxybenzoyl hexyl benzoate (labeling name, INCI: Diethylamino Hydroxybenzoyl Hexyl Benzoate), oxybenzone-6 (labeling name, INCI: Benzophenone-6), oxybenzone-9 (labeling name, INCI: Benzophenone-9), oxybenzone-1 (labeling name, INCI: Benzophenone-1), polysilicone-15 (INCI), dimethoxybenzylidene dioxoimidazolidine octyl propionate (labeling name, INCI: Ethylhexyl Dimethoxybenzylidene Dioxoimidazolidine Propionate), oxybenzone-2 (labeling name, INCI: Benzophenone-2), terephthalylidene dicamphor sulfonic acid (labeling name, INCI: Terephthalylidene Dicamphor Sulfonic Acid), ethylhexyl triazone (INCI), methylbis(trimethylsiloxy)silyl isopentyl trimethoxycinnamate (labeling name, INCI: Isopentyl Trimethoxycinnamate Trisiloxane), drometrizole trisiloxane (INCI), dimethyl PABA ethylhexyl (labeling name, INCI: Ethylhexyl Dimethyl PABA), isopropyl para-methoxycinnamate (labeling name, INCI: Isopropyl Methoxycinnamate), ethylhexyl methoxycinnamate (labeling name, INCI: Ethylhexyl Methoxycinnamate), bisethylhexyloxyphenol methoxyphenyltriazine (labeling name, INCI: Bis-Ethylhexyloxyphenol Methoxyphenyl Triazine), oxybenzone-3 (labeling name, INCI: Benzophenone-3), oxybenzone-4 (labeling name, INCI: Benzophenone-4), oxybenzone-5 (labeling name, INCI: Benzophenone-5), phenylbenzimidazole sulfonic acid (labeling name, INCI: Phenylbenzimidazole Sulfonic Acid), methylene bisbenzotriazolyl tetramethylbutylphenol (labeling name, INCI: Methylene Bis-Benzotriazolyl Tetramethylbutylphenol), glyceryl ethylhexanoate dimethoxycinnamate (labeling name, INCI: Glyceryl Ethylhexanoate Dimethoxycinnamate), glyceryl PABA (labeling name, INCI: Glyceryl PABA), methyl diisopropylcinnamate (labeling name, INCI: Diisopropyl Methyl Cinnamate), cinoxate (INCI), ethylhexyl dimethoxybenzylidene dioxoimidazolidine propionate (labeling name, INCI: Ethylhexyl Dimethoxybenzylidene Dioxoimidazolidine Propionate), etc. Also, UVA absorbers, e.g., diethylaminohydroxybenzoyl hexyl benzoate (labeling name, INCI: Diethylamino Hydroxybenzoyl Hexyl Benzoate) and UVB absorbers, e.g., ethylhexyl methoxcinnamate (labeling name, INCI: Ethylhexyl Methoxycinnamate) may be used in combination. Also, each of them may be used in combination.

### (2) Aqueous component

The aqueous components are not particularly limited as long as they are commonly blended in cosmetics. Examples include water, lower alcohols of 2 to 5 carbon atoms such as ethanol (labeling name, INCI: Alcohol) and isopropanol (labeling name, INCI: Isopropyl Alcohol); and sucrose alcohols such as sorbitol (INCI), maltose (INCI) and xylitol (INCI). Also included are plyhydric alcohols such as BG (labeling name, INCI: Butyl Glycol), PG (labeling name, INCI: Propylene Glycol), DPG (labeling name, INCI: Dipropylene Glycol), pentylene glycol (INCI), 1,10-decanediol (INCI), octanediol (INCI), 1,2-hexanediol (INCI), erythritol (INCI), glycerin (INCI), diglycerin (INCI), and polyethylene glycol; and humectants such as glucose (INCI), glyceryl glucoside (INCI), betaine (INCI), sodium chondroitin sulfate (labeling name, INCI: Sodium Chondroitin Sulfate), PCA-Na (labeling name, INCI: Sodium PCA), Methyl Gluceth-10 (INCI), Methyl Gluceth-20 (INCI), hyaluronic acid, egg yolk lecithin, soybean lecithin, phosphatidylcholine, phosphatidylethanolamine, phosphatidylserine, phosphatidylglycerol, phosphatidylinositol, and sphingolipid.

### (3) Surfactant other than component (A)

The surfactant may be nonionic, anionic, cationic or ampholytic. The surfactant is not particularly limited as long as it is other than component (A) and commonly blended in cosmetics. Of the surfactants, preferred are partially crosslinked polyether-modified silicones, straight or branched polyoxyethylene-modified organopolysiloxanes, straight or branched polyoxyethylenepolyoxypropylene-modified organopolysiloxanes, straight or branched polyoxyethylene/alkyl-co-modified organopolysiloxanes, and straight or branched polyoxyethylenepolyoxypropylene/alkyl-co-modified organopolysiloxanes. In these surfactants, the content of hydrophilic polyoxyethylene groups or polyoxyethylenepolyoxypropylene groups is preferably 10 to 70% by weight of the molecule.

Examples of the partially crosslinked polyether-modified silicone include (dimethicone/(PEG-10/15)) crosspolymer (INCI), (PEG-15/lauryl dimethicone) crosspolymer (INCI), (PEG-10/lauryl dimethicone) crosspolymer (INCI), and (PEG-15/lauryl polydimethylsiloxyethyl dimethicone) crosspolymer (INCI). On use of partially crosslinked polyether-modified silicone, it is preferred that in a composition consisting of the crosslinked organopolysiloxane and an oil which is liquid at 25°C, the crosslinked organopolysiloxane swell with the liquid oil as a result of being impregnated with an amount of more than its own weight of the liquid oil.

Suitable liquid oils include liquid silicones, hydrocarbon oils, ester oils, natural animal and plant oils, semi-synthetic oils and fluorochemical oils, all exemplified above as component (B) or (1) the oil other than component (B). Illustrative examples include cyclopentasiloxane (INCI), dimethicone (INCI), mineral oil (INCI), isododecane (INCI), isohexadecane (INCI), triethylhexanoin (INCI), isotridecyl isononanoate (labeling name, INCI: Isotridecyl Isononanoate), and squalane (INCI). Commercially available examples of the crosslinked organopolysiloxane which is swollen with the liquid oil include KSG-210, KSG-240, KSG-270, KSG-310, KSG-320, KSG-330, KSG-340, KSG-320Z, and KSG-350Z, from Shin-Etsu Chemical Co., Ltd.

Examples of the surfactant other than the crosslinked organopolysiloxane include PEG-11 methyl ether dimethicone (INCI), PEG/PPG-20/22 butyl ether dimethicone (INCI), PEG-3 dimethicone (INCI), PEG-10 dimethicone (INCI), PEG-9 polydimethylsiloxyethyl dimethicone (INCI), lauryl PEG-9 polydimethylsiloxyethyl dimethicone (INCI), and cetyl PEG/PPG-10/1 dimethicone (INCI). Commercially available examples include KF-6011, KF-6011P, KF-6012, KF-6015, KF-6017, KF-6043, KF-6028, KF-6038, and KF-6048, from Shin-Etsu Chemical Co., Ltd.

For any of the foregoing surfactants, the content thereof, inclusive of component (A), is preferably 0.1 to 20% by weight of the overall cosmetic composition. A surfactant content of at least 0.1% by weight enables to fully exert the function of dispersion or emulsification whereas a surfactant content of up to 20% by weight eliminates the risk that the cosmetic composition has a sticky feeling on use. For the purpose of maintaining the cosmetic composition water resistant, the surfactant preferably has a HLB value of 2 to 14.5 although the HLB is not particularly limited.

### (4) Powder

Suitable powders include coloring pigments, inorganic powders, metal powders, organic powders, and inorganic/organic composite powders, which are exemplified below.

### • Coloring pigments

The coloring pigment is not particularly limited as long as it is commonly used in cosmetics for the purpose of coloring cosmetic compositions. Examples include red iron oxide (labeling name, INCI: Iron Oxides), yellow iron oxide (labeling name, INCI: Iron Oxides), white titanium oxide (labeling name, INCI: Titanium Dioxide), black iron oxide (labeling name, INCI: Iron Oxides), Ultramarine (labeling name, INCI: Ultramarines), Prussian blue (labeling name, INCI: Ferric Ferrocyanide, Ferric Ammonium Ferrocyanide), manganese violet (labeling name, INCI: Manganese Violet), cobalt titanate (labeling name, INCI: Cobalt Titanium Oxide), chromium hydroxide (labeling name, INCI: Chromium Hydroxide Green), chromium oxide (labeling name, INCI: Chromium Oxide Greens), Al/cobalt oxide (labeling name, INCI: Cobalt Aluminum Oxide), cobalt titanate (labeling name, INCI: Cobalt Titanium Oxide), fired titanium/titanium oxide (labeling name, INCI: Titanium/Titanium Dioxide), Li/cobalt titanate (labeling name, INCI: Lithium Cobalt Titanate), cobalt titanate (labeling name, INCI: Cobalt Titanium Oxide), sintered iron oxide/titanium oxide (labeling name), composites doped with a different metal such as iron oxide-doped titanium oxide (labeling name, INCI: Iron Oxides, Titanium Dioxide), titanium nitride (labeling name, INCI: Titanium Nitride), ferrous hydroxide (labeling name, INCI: Iron Hydroxide), inorganic brown pigments such as γ-iron oxide, inorganic yellow pigments such as loess, colored pigments such as lake form of tar dyes and lake form of natural dyes. Any of the foregoing may be used.

The coloring pigment may take any shape including spherical, generally spherical, bar, spindle, petal, strip, and irregular shapes. Its geometry is not particularly limited as long as a color can be imparted to a cosmetic composition.

### • Inorganic powder

Examples of the inorganic powder include microparticles of zirconium oxide (labeling name, INCI: Zirconium Dioxide), zinc oxide (labeling name, INCI: Zinc Oxide), cerium oxide (labeling name, INCI: Cerium Oxide), magnesium oxide (labeling name, INCI: Magnesium Oxide), barium sulfate (labeling name, INCI: Barium Sulfate), calcium sulfate (labeling name, INCI: Calcium Sulfate), magnesium sulfate (labeling name, INCI: Magnesium Sulfate), calcium carbonate (labeling name, INCI: Calcium Carbonate), magnesium carbonate (labeling name, INCI: Magnesium Carbonate), talc (INCI), mica (INCI), kaolin (INCI), synthetic fluorphlogopite (labeling name, INCI: Synthetic Fluorphlogopite), synthetic gold mica iron (labeling name), black mica (labeling name, INCI: Biotite), potassium silicate (labeling name, INCI: Potassium Silicate), silica (INCI), aluminum silicate (labeling name, INCI: Aluminum Silicate), magnesium silicate (labeling name, INCI: Magnesium Silicate), Al/Mg silicate (labeling name, INCI: Magnesium Aluminum Silicate), calcium silicate (labeling name, INCI: Calcium Silicate), Al/Ca/Na silicate (labeling name, INCI: Aluminum Calcium Sodium Silicate), Li/Mg/Na silicate (labeling name, INCI: Lithium Magnesium Sodium Silicate), Na/Mg silicate (labeling name, INCI: Sodium Magnesium Silicate), Ca/Al borosilicate (labeling name, INCI: Calcium Aluminum Borosilicate), Ca/Na borosilicate (labeling name, INCI: Calcium Sodium Borosilicate), hydroxyapatite (INCI), bentonite (INCI), montmorillonite (INCI), hectorite (INCI), zeolite (INCI), alumina (INCI), Al hydroxide (labeling name, INCI: Aluminum Hydroxide), boron nitride (labeling name, INCI: Boron Nitride), and glass (labeling name, INCI: Glass).

Also, examples of the inorganic coloring pearly pigment include pearly agents such as mica (INCI) coated with titanium oxide (labeling name, INCI: Titanium dioxide), and synthetic fluorphlogopite (labeling name, INCI: Synthetic Fluorphlogopite) coated with titanium oxide (labeling name, INCI: Titanium Dioxide); and pearly pigments such as bismuth oxychloride (labeling name, INCI: Bismuth Oxychloride), bismuth oxychloride (labeling name, INCI: Bismuth Oxychloride) coated with titanium oxide (labeling name, INCI: Titanium Dioxide), talc (labeling name, INCI: Talc) coated with titanium oxide (labeling name, INCI: Titanium Dioxide), fish scale flakes (labeling name), and coloring mica coated with titanium oxide (labeling name, INCI: Titanium Dioxide), which may either be untreated or have undergone well-known surface treatment commonly used for cosmetics.

### • Metal powder

Examples of the metal powder include metal microparticles such as aluminum (labeling name, INCI: Aluminum, Aluminum Powder), copper (labeling name, INCI: Copper Powder), silver (labeling name, INCI: Silver Powder), and gold (INCI: Gold).

### • Organic powder

Examples of the organic powder include silicone, polyamide, polyacrylic acid-acrylate, polyester, polyethylene (INCI), polypropylene (INCI), polystyrene (INCI), styrene-acrylic acid copolymers, divinylbenzene-styrene copolymers, polyurethane, vinyl resins, urea resins, melamine resins, benzoguanamine, polymethyl benzoguanamine, tetrafluoroethylene, polymethyl methacrylate, cellulose (INCI), silk (INCI), nylon (labeling name), phenolic resins, epoxy resins, polycarbonate, etc. in powder form.

In particular, as the silicone, silicone resin particles, e.g., polymethylsilsesquioxane (INCI), silicone rubber powder, and silicone resin-coated silicone rubber powder, (vinyl dimethicone/methicone silsesquioxane) crosspolymer (labeling name, INCI: Vinyl Dimethicone/Methicone Silsesquioxane Crosspolymer), (diphenyl dimethicone/vinyldiphenyl dimethicone/silsesquioxane) crosspolymer (labeling name, INCI: Diphenyl Dimethicone/Vinyl Diphenyl Dimethicone/Silsesquioxane Crosspolymer), polysilicone-1 crosspolymer (INCI), and polysilicone-22 (INCI). Commercially available examples of the silicone powder include KMP-590, KMP-591, KMP-592, KMP-597, KMP-598, KSP-100, KSP-101, KSP-102, KSP-105, KSP-300, KSP-411, KSP-441, KM-9729, and KM-440 from Shin-Etsu Chemical Co., Ltd.

Metal soaps are also included, examples of which include zinc stearate (labeling name, INCI: Zinc Stearate), Al stearate (labeling name, INCI: Aluminum Stearate), Ca stearate (labeling name, INCI: Calcium Stearate), Mg stearate (labeling name, INCI: Magnesium Stearate), zinc myristate (labeling name, INCI: Zinc Myristate), Mg myristate (labeling name, INCI: Magnesium Myristate), Zn/Na cetylphosphate (labeling name, INCI: Sodium Zinc Cetyl Phosphate), K cetylphosphate (labeling name, INCI: Potassium Cetyl Phosphate) in powder form.

Also included are organic colorants, examples of which include tar dyes, specifically Red #3, Red #104(1) (labeling name, INCI: Red 28, Red 28 Lake), Red #106, Red #201 (labeling name, INCI: Red 6), Red #202 (labeling name, INCI: Red 7), Red #204, Red #205, Red #220 (labeling name, INCI: Red 34), Red #226 (labeling name, INCI: Red 30), Red #227 (labeling name, INCI: Red 33, Red 33 Lake), Red #228 (labeling name, INCI: Red 36), Red #230(1) (labeling name, INCI: Red 22, Red 22 Lake), Red #230(2) (labeling name), Red #401 (labeling name), Red #505 (labeling name), Yellow #4 (labeling name, INCI: Yellow 5), Yellow #5 (labeling name, INCI: Yellow 6, Yellow 6 Lake), Yellow #202(1) (labeling name, INCI: Yellow 8), Yellow #203 (labeling name, INCI: Yellow 10, Yellow 10 Lake), Yellow #204 (labeling name, INCI: Yellow 11), Yellow #401, Blue #1 (labeling name, INCI: Blue 1, Blue 1 Lake), Blue #2, Blue #201, Blue #205 (labeling name, INCI: Blue 4), Blue #404 (labeling name), Green #3 (labeling name, INCI: Green 3, Green 3 Lake), Green #201 (labeling name, INCI: Green 5), Green #202 (labeling name, INCI: Green 6), Green #204 (labeling name, INCI: Green 8), Green #205 (labeling name), Orange #201 ((labeling name, INCI: Orange 5), Orange #203 (labeling name, INCI: Pigment Orange 5), Orange #204 (labeling name), Orange #205 (labeling name, INCI: Orange 4, Orange 4 Lake), Orange #206 (labeling name, INCI: Orange 10), Orange #207 (labeling name, INCI: Orange 11); and natural dyes, specifically cochineal (INCI), laccaic acid (labeling name, INCI: Laccaic Acid), safflower red (labeling name, INCI: Carthamus Tinctorius (Safflower) Flower Extract), red root extract (labeling name, INCI: Lithospermum Officinale Root Extract), gardenia yellow (labeling name), and gardenia blue (labeling name, INCI: Hydrolyzed Gardenia Florida Extract).

### • Inorganic/organic composite powder

Exemplary of the inorganic/organic composite powder is a composite powder obtained by coating an inorganic powder on the surface with an organic powder by any well-known method.

The above-mentioned powders may be used as having been treated on the surface of particles. The surface treating agent capable of imparting hydrophobicity is preferred from the aspect of water resistance of a cosmetic composition. The hydrophobizing agent is not particularly limited. Exemplary treating agents include silicone treating agents, waxes, paraffins, organic fluorine compounds such as perfluoroalkyl phosphate salts, surfactants, amino acids such as N-acylglutamic acid, and metal soaps such as aluminum stearate and magnesium myristate.

Among others, the silicone treating agents are preferably used. Included are silanes or silylating agents such as triethoxycaprylylsilane (INCI); silicone oils such as dimethicone (INCI), methicone (INCI), hydrogendimethicone (INCI), triethoxysilylethyl polydimethylsiloxyethyl dimethicone (INCI), triethoxysilylethyl polydimethylsiloxyethylhexyl dimethicone (INCI), and acrylate/tridecyl acrylate/triethoxysilyl-propyl methacrylate/dimethicone methacrylate copolymers (labeling name, INCI: Acrylate/Tridecyl Acrylate/Triethoxysilylpropyl Methacrylate/Dimethicone Methacrylate Copolymer).

Examples of the silicone treating agent include AES-3083, KF-99P, KF-9901, KF-9908, KF-9909, KP-574, and KP-541, from Shin-Etsu Chemical Co., Ltd.

The surface hydrophobizing agent may be used alone or in admixture. Examples of the surface treated coloring pigment include KTP-09 series, especially KTP-09W, KTP-09R, KTP-09Y and KTP-09B from Shin-Etsu Chemical Co., Ltd.

### (5) Composition consisting of crosslinked organopolysiloxane and an oil which is liquid at room temperature

In the composition consisting of crosslinked organopolysiloxane and an oil which is liquid at room temperature, it is preferred that the crosslinked organopolysiloxane swell with the liquid oil as a result of being impregnated with an amount of more than its own weight of the liquid oil. Suitable liquid oils include liquid silicone oils, hydrocarbon oils, ester oils, natural animal and plant oils, semi-synthetic oils and fluorochemical oils, all exemplified above as component (B) or (1) the oil as optional component. Illustrative examples include cyclopentasiloxane (INCI), dimethicone (INCI), mineral oil (INCI), isododecane (INCI), isohexadecane (INCI), triethylhexanoin (INCI), isotridecyl isononanoate (labeling name, INCI: Isotridecyl Isononanoate), and squalane (INCI).

Unlike component (A) and component (3) mentioned above, component (5) is a compound having neither polyether nor polyglycerin structure in its molecular structure. Examples include dimethicone/vinyl dimethicone crosspolymer (INCI), dimethicone/phenyl vinyl dimethicone crosspolymer (INCI), vinyl dimethicone/lauryl dimethicone crosspolymer (INCI), and lauryl polydimethylsiloxyethyl dimethicone/bisvinyl dimethicone crosspolymer (INCI). Commercially available examples of the composition consisting of crosslinked organopolysiloxane and an oil which is liquid at room temperature include KSG-15, KSG-1510, KSG-16, KSG-1610, KSG-19, KSG-016F, KSG-18A, KSG-41A, KSG-42A, KSG-43, KSG-44, KSG-042Z, KSG-045Z, and KSG-048Z from Shin-Etsu Chemical Co., Ltd.

### (6) Film-forming agent

The film-forming agent is blended for the purpose of further maintaining the effect-sustainability of the cosmetic composition. A silicone-based agent is preferred from the aspect of imparting water repellency though the agent is not limited thereto. Specifically use may be made of trimethylsiloxysilicic acid, acrylic-silicone film formers, silicone-modified norbornene, silicone-modified pullulan, and silicone-modified polyvinyl alcohol.

Examples of the film-forming agent in the form of silicone-based agent herein include trimethylsiloxysilicic acid (labeling name, INCI: Trimethylsiloxysilicate), acrylate/dimethicone copolymer (INCI), norbornene/tris-(trimethylsiloxy)silylnorbornene copolymer (INCI), and pullulan tri(trimethylsiloxy)silylpropyl carbamide (labeling name, INCI: Trimethylsiloxysilylcarbamoyl Pullulan).

The film-forming agent may be previously dissolved in an oil which is liquid at room temperature before it is blended in the cosmetic composition. Suitable liquid oils used herein include liquid silicone oils, hydrocarbon oils, ester oils, natural animal and plant oils, semi-synthetic oils, and fluorochemical oils, all included in component (B) or (1) the oil as optional component. Illustrative examples include cyclopentasiloxane (INCI), dimethicone (INCI), mineral oil (INCI), isododecane (INCI), isohexadecane (INCI), triethylhexanoin (INCI), isotridecyl isononanoate (labeling name, INCI: Isotridecyl Isononanoate), squalane (INCI), and butyl acetate (labeling name, INCI: Butyl Acetate).

Commercially available examples of the silicone film-forming agent include KF-7312J, KP-545, KP-549, KP-543, NBN-30-ID, TSPL-30-ID, and TSPL-30-D5 from Shin-Etsu Chemical Co., Ltd.

### (7) UV absorbing/scattering agent

Suitable UV absorbing/scattering agents are particles capable of absorbing and scattering UV, including microparticulate titanium oxide, microparticulate iron-containing titanium oxide, microparticulate zinc oxide, microparticulate cerium oxide, and composites thereof. Dispersions having UV absorbing/scattering particles previously dispersed in an oil are also acceptable. Suitable oils include liquid silicone oils, hydrocarbon oils, ester oils, natural animal and plant oils, semi-synthetic oils and fluorochemical oils, all exemplified above as component (B) or (1) the oil other than component (B). Illustrative examples include cyclopentasiloxane (INCI), dimethicone (INCI), mineral oil (INCI), isododecane (INCI), isohexadecane (INCI), triethylhexanoin (INCI), isotridecyl isononanoate (labeling name, INCI: Isotridecyl Isononanoate), and squalane (INCI). Commercially available examples of the dispersion having UV absorbing/scattering particles previously dispersed in an oil include SPD series, specifically SPD-T5, SPD-Z5, SPD-T6, SPD-Z6, and SPD-T7, all from Shin-Etsu Chemical Co., Ltd.

### (8) Other additives

Other additives include oil-soluble gelling agents, preservatives, antibacterial agents, antiperspirants, fragrances, salts, antioxidants, acidity regulators, chelating agents, refreshing agents, anti-inflammatory agents, skin modifying ingredients (e.g., brightening or whitening agents, cell activators, anti-skin-roughening agents, blood flow enhancing agents, skin astringents, antiseborrheic agents), vitamins, amino acids, nucleic acids, hormones, and clathrate compounds.

### • Oil-soluble gelling agent

Exemplary oil-soluble gelling agents include metal soaps such as aluminum stearate, magnesium stearate and zinc myristate; amino acid derivatives such as lauroyl glutamic acid (labeling name, INCI: Lauroyl Glutamic Acid ) and α,γ-di-n-butylamine; dextrin fatty acid esters such as dextrin palmitate (labeling name, INCI: Dextrin Palmitate), dextrin isostearate (labeling name, INCI: Dextrin Isostearate), dextrin myristate (labeling name, INCI: Dextrin Myristate), inulin stearate (labeling name, INCI: Stearoyl Inulin), dextrin palmitate/ethylhexanoate (labeling name, INCI: Dextrin Palmitate/Ethylhexanoate); sucrose fatty acid esters such as sucrose palmitate and sucrose stearate; fructooligosaccharide fatty acid esters such as fructooligosaccharide stearate and fructooligosaccharide 2-ethylhexanoate; benzylidene derivatives of sorbitol such as monobenzylidene sorbitol and dibenzylidene sorbitol; organo-modified clay minerals such as disteardimonium hectorite (INCI), stearalkonium hectorite (INCI), and hectorite; and stearalkonium bentonite (INCI).

### • Preservative/antibacterial agents

Suitable preservatives and antibacterial agents include alkyl p-hydroxybenzoates, benzoic acid, sodium benzoate, sorbic acid, potassium sorbate, phenoxyethanol, imidazolidinyl urea, salicylic acid, isopropyl methyl phenol, phenol, p-chloro-m-cresol, hexachlorophene, benzalkonium chloride, chlorohexidine chloride, trichloroarbanilide, iodopropynyl butylcarbamate, polylysine, photosensitizers, silver, and plant extracts.

### • Antiperspirant

Examples of the antiperspirant include aluminum halohydrates such as aluminum chlorohydrate, aluminum halides such as aluminum chloride, aluminum allantoinate, tannic acid, persimmon, aluminum potassium sulfate, zinc oxide, zinc p-phenolsulfonate, aluminum potassium sulfate, aluminum zirconium tetrachlorohydrate, and aluminum zirconium trichlorohydrex glycine. The preferred components which exert a higher effect include aluminum halohydrates, aluminum halides, and complexes or mixtures thereof with zirconium oxyhalides and zirconium hydroxyhalides (e.g., aluminum zirconium tetrachlorohydrate and aluminum zirconium trichlorohydrex glycine.

### • Fragrance

Fragrances include natural fragrances and synthetic fragrances. Suitable natural fragrances include plant fragrances separated from flowers, leaves, stems, and pericarps, and animal fragrances such as musk and civet. Suitable synthetic fragrances include hydrocarbons such as monoterpene; alcohols such as aliphatic alcohols and aromatic alcohols; aldehydes such as terpene aldehydes and aromatic aldehydes; ketones such as alicyclic ketones; esters such as terpene esters; lactones; phenols; oxides; nitrogen-containing compounds; and acetals.

### • Salt

Salts include inorganic salts, organic acid salts, amine salts, and amino acid salts. Exemplary inorganic salts include sodium, potassium, magnesium, calcium, aluminum, zirconium and zinc salts of inorganic acids such as hydrochloric acid, sulfuric acid, carbonic acid and nitric acid. Exemplary organic acid salts include salts of organic acids such as acetic acid, dehydroacetic acid, citric acid, malic acid, succinic acid, ascorbic acid, and stearic acid. Exemplary amine salts and amino acid salts include salts of amines such as triethanol amine, and salts of amino acids such as glutamic acid. Also useful are hyaluronic acid, salts such as chondroitin sulfate, aluminum zirconium glycine complex, and acid-alkali neutralized salts used in cosmetic formulations.

### • Antioxidant

Examples of the antioxidant include, but are not particularly limited to, carotenoid, ascorbic acid and salts thereof, ascorbyl stearate, tocopherol acetate, p-t-butylphenol, butyl hydroxyanisole, dibutyl hydroxytoluene, phytic acid, ferulic acid, thiotaurine, hypotaurine, sulfites, erythorbic acid and salts thereof, chlorogenic acid, epicatechin, epigallocatechin, epigallocatechin gallate, apigenin, kaempferol, myricetin, and quercetin. The antioxidants may be used alone or in admixture.

### • Acidity regulator

Suitable acidity regulators include lactic acid, citric acid, glycolic acid, succinic acid, tartaric acid, dl-malic acid, potassium carbonate, sodium hydrogencarbonate, and ammonium hydrogencarbonate.

### • Chelating agent

Suitable chelating agents include alanine, sodium EDTA, sodium polyphosphate, sodium metaphosphate, and phosphoric acid.

### • Refreshing agent

Suitable refreshing agents include L-menthol, camphor and menthyl lactate.

### • Anti-inflammatory agent

Suitable anti-inflammatory agents include allantoin, glycyrrhizinic acid and salts thereof, glycyrrhetinic acid, stearyl glycyrrhetinate, tranexamic acid, and azulene.

### • Skin modifying ingredient

Suitable skin modifying ingredients include brightening or whitening agents such as placenta extract, arbutin, glutathione, and Saxifraga Sarmentosa Extract; cell activators such as royal jelly, photosensitizers, cholesterol derivatives, and bovine blood extracts; anti-skin-roughening agents; blood flow enhancing agents such as vanillylamide nonanoate, benzyl nicotinate, β-butoxyethyl nicotinate, capsaicin, zingerone, cantharides tincture, ichthammol, caffeine, tannic acid, α-borneol, tocopherol nicotinate, inositol hexanicotinate, cyclandelate, cinnarizine, tolazoline, acetylcholine, verapamil, cepharanthine, and γ-oryzanol; skin astringents such as zinc oxide and tannic acid; and antiseborrheic agents such as sulfur and thianthol.

### • Vitamin

Suitable vitamins include vitamin A family such as vitamin A oil, retinol, retinol acetate, retinol palmitate; vitamin B family, specifically vitamin B2 family such as riboflavin, riboflavin butyrate, and flavin adenine nucleotide, vitamin B6 family such as pyridoxine hydrochloride, pyridoxine dioctanoate, pyridoxine tripalmitate, vitamin B12 and derivatives thereof, vitamin B15 and derivatives thereof; vitamin C family such as L-ascorbic acid, L-ascorbic acid dipalmitic acid ester, L-ascorbic acid-2-sodium sulfate, and dipotassium L-ascorbic acid phosphoric acid diester; vitamin D family such as ergocalciferol and cholecalciferol; vitamin E family such as α-tocopherol, β-tocopherol, γ-tocopherol, dl-α-tocopherol acetate, dl-α-tocopherol nicotinate, and dl-α-tocopherol succinate; nicotinic acids such as nicotinic acid, benzyl nicotinate, and nicotinic amide; vitamin H, vitamin P, pantothenic acids such as calcium pantothenate, D-pantothenyl alcohol, pantothenyl ethyl ether, acetylpantothenyl ethyl ether; and biotin.

### • Amino acid

Suitable amino acids include glycine, valine, leucine, isoleucine, serine, threonine, phenylalanine, arginine, lysine, aspartic acid, glutamic acid, cystine, cysteine, methionine, and tryptophan.

### • Nucleic acid

Typical of the nucleic acid is deoxyribonucleic acid.

### • Hormone

Suitable hormones include estradiol and ethenyl estradiol.

### • Clathrate compound

Typical of the clathrate compound is cyclodextrin.

The inventive cosmetic composition may be in powder, liquid and solid forms. Main types of formulation include liquid, cream, aerosol, ointment, emulsified solid, stick, emulsified stick, etc.

### EXAMPLES

Examples and Comparative Examples are shown below by way of illustration and not by way of limitation. In Examples and Comparative Examples, compositional percent (%) is by weight and parts are parts by weight (pbw). The amount of product name is the amount of the product blended. Component (B) used herein all had a solubility of less than 1 g in 100 g of purified water at 25°C.

### [Examples and Comparative Examples (Surfactant composition-1)]

Surfactant compositions of the formulation shown in Tables 1 and 2 were prepared.

### (Preparation method)

The composition was prepared by metering selected components and mixing them.

The surfactant compositions were evaluated as follows. The results are also shown in Tables.

### [Viscosity]

A 30-ml vial was used. The viscosity was measured at 25°C by the method described in JIS K7117-1: 1999 using a Brookfield rotational viscometer (TVB-10 model, Toki Sangyo Co., Ltd.).

### [Transparency]

A 30-ml vial was observed with naked eyes under 25°C conditions. The composition was rated "○" in dissolved transparent state, "△" in dissolved semitransparent state, and "×" in separated state. Rated with "△" or above ("△" or "O") were judged "Pass".

**[Table 1]**

| Formulation (%) | | Example | | | | | | | | | | | |
|---|---|---|---|---|---|---|---|---|---|---|---|---|---|
| | | 1 | 2 | 3 | 4 | 5 | 6 | 7 | 8 | 9 | 10 | 11 | 12 |
| (A) | Polyglyceryl-3 polydimethylsiloxyethyl dimethicone (*1) (viscosity 3,392 mPa·s, 4 hydroxy groups on average) | 90 | 90 | | | | | | | | | | |
| | Lauryl polyglyceryl-3 polydimethylsiloxyethyl dimethicone (*2) (viscosity 3,110 mPa·s, 4 hydroxy groups on average) | | | 90 | 90 | 90 | 90 | 90 | 90 | | | | |
| | Polyglyceryl-3 disiloxane dimethicone (*3) (viscosity 42,100 mPa·s, 4 hydroxy groups on average) | | | | | | | | | 90 | | | |
| | Polyglyceryl-2 isostearate (viscosity 5,650 mPa·s, 3 hydroxy groups on average) | | | | | | | | | | 90 | 90 | |
| | Polyglyceryl-10 laurate (viscosity 92,500 mPa·s, 11 hydroxy groups on average) | | | | | | | | | | | | 90 |
| (B) | Hexyldecanol (viscosity 30.9 mPa·s) | | | 10 | | | | | | | | | |
| | Isostearyl alcohol (viscosity 45.7 mPa·s) | 10 | | | 10 | | | | | 10 | 10 | | 10 |
| | Octyl dodecanol (viscosity 51.1 mPa·s) | | | | | | | | | | | 10 | |
| | Decyl tetradecanol (viscosity 54.5 mPa·s) | | 10 | | | 10 | | | | | | | |
| | Oleic acid (viscosity 30.1 mPa·s) | | | | | | 10 | | | | | | |
| | Diisostearyl malate (viscosity 2,963 mPa·s) | | | | | | | 10 | | | | | |
| | Polyglyceryl-2 triisostearate (viscosity 357 mPa·s) | | | | | | | | 10 | | | | |
| Total | | 100 | 100 | 100 | 100 | 100 | 100 | 100 | 100 | 100 | 100 | 100 | 100 |
| Viscosity (mPa·s) | | 568 | 1,243 | 605 | 677 | 833 | 961 | 1,379 | 1,502 | 15,000 | 2,230 | 2,300 | 45,800 |
| Transparency | | ○ | △ | ○ | ○ | ○ | ○ | ○ | △ | ○ | ○ | ○ | △ |

| | | | | | | | | | | | | | |
|---|---|---|---|---|---|---|---|---|---|---|---|---|---|
| (*1) KF-6104 by Shin-Etsu Chemical Co., Ltd. (*2) KF-6105 by Shin-Etsu Chemical Co., Ltd. (*3) KF-6100 by Shin-Etsu Chemical Co., Ltd. | | | | | | | | | | | | | |

**[Table 2]**

| Formulation (%) | | Comparative Example | | | | | | | | | |
|---|---|---|---|---|---|---|---|---|---|---|---|
| | | 1 | 2 | 3 | 4 | 5 | 6 | 7 | 8 | 9 | 10 |
| (A) | Polyglyceryl-3 polydimethylsiloxyethyl dimethicone (*1) (viscosity 3,392 mPa·s, 4 hydroxy groups on average) | 90 | 90 | | | | | | | | |
| | Lauryl polyglyceryl-3 polydimethylsiloxyethyl dimethicone (*2) (viscosity 3,110 mPa·s, 4 hydroxy groups on average) | | | 90 | 90 | 90 | 90 | | | | |
| | Polyglyceryl-3 disiloxane dimethicone (*3) (viscosity 42,100 mPa·s, 4 hydroxy groups on average) | | | | | | | 90 | | | |
| | Polyglyceryl-2 isostearate (viscosity 5,650 mPa·s, 3 hydroxy groups on average) | | | | | | | | 90 | | |
| | Polyglyceryl-10 laurate (viscosity 92,500 mPa·s, 11 hydroxy groups on average) | | | | | | | | | 90 | |
| (A) Comparison | PEG-10 dimethicone (*4) (viscosity 542 mPa·s, 1 hydroxy group on average) | | | | | | | | | | 90 |
| (B) | Isostearyl alcohol (viscosity 45.7 mPa·s) | | | | | | | | | | 10 |
| (B) Comparison | Dimethicone (viscosity 6.0 mPa·s) | 10 | | | | | | | | | |
| | Mineral oil (viscosity 17.2 mPa·s) | | | 10 | | | | | | | |
| | Isostearyl isostearate (viscosity 34.7 mPa·s) | | 10 | | 10 | | | 10 | 10 | 10 | |
| | Polyglyceryl-2 diisostearate (viscosity 759 mPa·s) | | | | | 10 | | | | | |
| | Polyglyceryl-2 tetraisostearate (viscosity 253 mPa·s) | | | | | | 10 | | | | |
| Total | | 100 | 100 | 100 | 100 | 100 | 100 | 100 | 100 | 100 | 100 |
| Viscosity (mPa·s) | | 2,407 | 2,191 | 1,851 | 1,686 | 1,781 | 2,175 | 31,800 | 3,250 | 74,300 | 313 |
| Transparency | | ○ | △ | ○ | ○ | ○ | △ | △ | ○ | △ | ○ |

| | | | | | | | | | | | |
|---|---|---|---|---|---|---|---|---|---|---|---|
| (*1) KF-6104 by Shin-Etsu Chemical Co., Ltd. (*2) KF-6105 by Shin-Etsu Chemical Co., Ltd. (*3) KF-6100 by Shin-Etsu Chemical Co., Ltd. (*4) KF-6017 by Shin-Etsu Chemical Co., Ltd. | | | | | | | | | | | |

For the surfactant compositions of Examples and Comparative Examples, a percent lowering of their viscosity is shown in Table 3.

### [Viscosity lowering]

A percent lowering of the viscosity of the surfactant composition from the viscosity of component (A) was computed.

**[Table 3]**

| | Viscosity (mPa·s) | | Viscosity lowering (%) |
|---|---|---|---|
| | Component (A) | Composition | |
| Example 1 | 3,392 | 568 | 16.7 |
| Example 2 | 3,392 | 1,243 | 36.6 |
| Example 3 | 3,110 | 605 | 19.5 |
| Example 4 | 3,110 | 677 | 21.8 |
| Example 5 | 3,110 | 833 | 26.8 |
| Example 6 | 3,110 | 961 | 30.9 |
| Example 7 | 3,110 | 1,379 | 44.3 |
| Example 8 | 3,110 | 1,502 | 48.3 |
| Example 9 | 42,100 | 15,000 | 35.6 |
| Example 10 | 5,650 | 2,230 | 39.5 |
| Example 11 | 5,650 | 2,300 | 40.7 |
| Example 12 | 92,500 | 45,800 | 49.5 |
| Comparative Example 1 | 3,392 | 2,407 | 71.0 |
| Comparative Example 2 | 3,392 | 2,191 | 64.6 |
| Comparative Example 3 | 3,110 | 1,851 | 59.5 |
| Comparative Example 4 | 3,110 | 1,686 | 54.2 |
| Comparative Example 5 | 3,110 | 1,781 | 57.3 |
| Comparative Example 6 | 3,110 | 2,175 | 69.9 |
| Comparative Example 7 | 42,100 | 31,800 | 75.5 |
| Comparative Example 8 | 5,650 | 3,250 | 57.5 |
| Comparative Example 9 | 92,500 | 74,300 | 80.3 |
| Comparative Example 10 | 542 | 313 | 57.7 |

It is demonstrated in Table 3 that the surfactant compositions of Examples 1 to 12 show an acceptable lowering of viscosity of the surfactant. As apparent from the results of Examples 6 to 8, this effect is not simply attributed to the viscosity of component (B). In Examples 1 to 5 which used polyglycerin-modified silicone as component (A) and branched saturated alcohol as component (B), a more outstanding viscosity lowering was obtained. As seen from Comparative Examples 1 to 9, when component (A) was combined with commonly used silicone or hydrocarbon oils, ester oil having a viscosity approximate to component (B), or different component (A), the viscosity lowering was insufficient. Comparative Example 10 using a surfactant having 1 hydroxy group per hydrophilic group failed to achieve an acceptable viscosity lowering even when combined with component (B). It is seen from the foregoing results that the combination of component (A) with component (B) is critical to the viscosity lowering.

### [Examples and Comparative Examples (cosmetic compositions)]

Cosmetic compositions of W/O type were prepared in accordance with the formulation shown in Table 4.

### (Preparation method)

A: Mixing component (1).
B: Mixing component (2).
C: Adding the mixture of B to the mixture of A and emulsifying to prepare a W/O type cream.

### [Evaluation of properties]

Ease of mixing in oil phase (or ease of metering) during the preparation of a cosmetic composition and the feeling on use (or ease of spreading) of the cosmetic composition were evaluated by a panel of 10 professional members. The result is an average of ratings of 10 panel members and rated according to the following judgment criterion. The results are also shown in Tables.

### [Evaluation criterion]

| | |
|---|---|
| 5 points: | excellent |
| 4 points: | good |
| 3 points: | mediocre |
| 2 points: | rather poor |
| 1 point: | poor |

After the average point was computed, the sample was rated in the O-× scale according to the following criterion.

### [Evaluation criterion]

| | |
|---|---|
| ⊚: | 4.5 or more |
| O: | from 3.5 to less than 4.5 |
| △: | from 2.5 to less than 3.5 |
| ×: | from 1.5 to less than 2.5 |
| ××: | less than 1.5 |

Rated with "△" or above were judged "Pass".

**[Table 4]**

| Formulation (%) | | Example | | | | Comparative Example | | |
|---|---|---|---|---|---|---|---|---|
| | | 13 | 14 | 15 | 16 | 11 | 12 | 13 |
| (1) | Surfactant composition of Example 1 | 4 | | | | | | |
| | Surfactant composition of Example 3 | | 4 | | | | | |
| | Surfactant composition of Comparative Example 6 | | | | | | | 4 |
| | (A) Polyglyceryl-3 polydimethylsiloxyethyl dimethicone (*1) | | | 3.6 | | 3.6 | | |
| | (A) Lauryl polyglyceryl-3 polydimethylsiloxyethyl dimethicone (*2) | | | | 3.6 | | 3.6 | |
| | (B) Isostearyl alcohol | | | 0.4 | 0.4 | | | |
| | Dimethicone (6 cs) | 19 | | 19 | | 19.4 | | |
| | Mineral oil | | 19 | | 19 | | 19.4 | 19 |
| (2) | Sodium chloride | 0.5 | 0.5 | 0.5 | 0.5 | 0.5 | 0.5 | 0.5 |
| | BG | 5 | 5 | 5 | 5 | 5 | 5 | 5 |
| | Water | 71.5 | 71.5 | 71.5 | 71.5 | 71.5 | 71.5 | 71.5 |
| Total | | 100.0 | 100.0 | 100.0 | 100.0 | 100.0 | 100.0 | 100.0 |
| Mixing | | ○ | ○ | △ | △ | △ | △ | △ |
| Feeling on use | | ○ | ○ | ○ | ○ | × | × | × |

| | | | | | | | | |
|---|---|---|---|---|---|---|---|---|
| (*1) KF-6104 by Shin-Etsu Chemical Co., Ltd. (*2) KF-6105 by Shin-Etsu Chemical Co., Ltd. | | | | | | | | |

As is evident from Table 4, the cosmetic compositions of Examples 13 to 16 gave a pleasant feeling on use. Especially, Examples 13 and 14 were so easy to mix that the cosmetic composition was readily prepared. The viscosity at 25°C of mixture (1) in Examples 15 and 16 was less than 50% of the viscosity (3,392 mPa·s) of component (A).

### [Examples and Comparative Examples (Surfactant composition-2)]

Surfactant compositions were prepared in accordance with the formulation shown in Table 5.

### (Preparation method)

The surfactant composition was prepared by metering ingredients into a beaker and mixing them.

The composition was evaluated for viscosity, transparency and viscosity lowering by the same methods as above. The results are also shown in Table.

**[Table 5]**

| Formulation (%) | Example | | | | | | Comp. Ex. |
|---|---|---|---|---|---|---|---|
| | 17 | 18 | 19 | 20 | 21 | 22 | 14 |
| (A) Lauryl polyglyceryl-3 polydimethylsiloxyethyl dimethicone (*1) (viscosity 3,110 mPa·s, 4 hydroxy groups on average) | 95 | 95 | 95 | | | | |
| (A) Polyglyceryl-3 disiloxane dimethicone (*2) (viscosity 42,100 mPa·s, 4 hydroxy groups on average) | | | | 80 | 80 | 80 | 80 |
| (B) Hexyldecanol (viscosity 30.9 mPa·s) | 5 | | | | | | |
| (B) Isostearyl alcohol (viscosity 45.7 mPa·s) | | 5 | | 20 | | | |
| (B) Octyldodecanol (viscosity 51.1 mPa·s) | | | 5 | | 20 | | |
| (B) Decyltetradecanol (viscosity 54.5 mPa·s) | | | | | | 20 | |
| (B) Isostearyl isostearate (viscosity 34.7 mPa·s) | | | | | | | 20 |
| Total | 100 | 100 | 100 | 100 | 100 | 100 | 100 |
| Viscosity (mPa·s) | 1,179 | 1,186 | 1,276 | 8,380 | 10,580 | 21,000 | 39,100 |
| Transparency | ○ | ○ | ○ | ○ | △ | △ | △ |
| Viscosity lowering (%) | 37.9 | 38.1 | 41.0 | 19.9 | 25.1 | 49.9 | 92.9 |

| | | | | | | | |
|---|---|---|---|---|---|---|---|
| (*1) KF-6105 by Shin-Etsu Chemical Co., Ltd. (*2) KF-6100 by Shin-Etsu Chemical Co., Ltd. | | | | | | | |

As evident from Table 6, the surfactant compositions of Examples 17 to 22 showed an acceptable lowering of surfactant viscosity even when the weight ratio of component (A) to component (B) was adjusted.

### [Example 23] W/O sunscreen cream

| Ingredient | | % |
|---|---|---|
| 1. | Cyclopentasiloxane | 30 |
| 2. | Squalane | 2.5 |
| 3. | Surfactant composition of Example 3 | 4.5 |
| 4. | Ethylhexyl methoxysuccinate | 7.5 |
| 5. | t-Butylmethoxydibenzoylmethane | 3 |
| 6. | Polysilicone-15 | 1 |
| 7. | Disteardimonium hectorite | 1 |
| 8. | Tocopherol acetate | 0.1 |
| 9. | Ethanol | 1 |
| 10. | Sodium citrate | 0.5 |
| 11. | Magnesium sulfate | 0.5 |
| 12. | Preservative | 0.3 |
| 13. | Water | 48.1 |
| Total | | 100.0 |

### (Preparation method)

A: Uniformly mixing ingredients 1 to 8.
B: Uniformly dissolving ingredients 9 to 12 in ingredient 13.
C: With stirring, slowly adding the solution of B to the mixture of A and emulsifying to prepare a W/O sunscreen cream.

The W/O sunscreen cream thus obtained showed a quite pleasant feeling on use, no changes with temperature and time, and good stability. Since the surfactant composition within the scope of the invention was used, the preparation of the cosmetic composition was easy.

### [Example 24] W/O creamy lipstick

| Ingredient | | % |
|---|---|---|
| 1. | Dextrin palmitate/ethylhexanoate (*1) | 9 |
| 2. | Cyclopentasiloxane | 53 |
| 3. | KP-545 (*2) | 5 |
| 4. | KSG-43 (*3) | 8 |
| 5. | Surfactant composition of Example 6 | 2 |
| 6. | BG | 5 |
| 7. | Water | 10.8 |
| 8. | Coloring agent | 1.2 |
| 9. | Titanium oxide-coated mica | 6 |
| Total | | 100.0 |

| | | |
|---|---|---|
| (*1) Rheopearl TT by Ciba Flour Milling Co., Ltd. (*2) Solution of cyclopentasiloxane 70% + acrylate/dimethicone copolymer 30%, by Shin-Etsu Chemical Co., Ltd. (*3) Mixture of triethylhexanoin 65-75% + vinyl dimethicone/lauryl dimethicone crosspolymer 25-35%, by Shin-Etsu Chemical Co., Ltd. | | |

### (Preparation method)

A: Adding ingredient 8 to a portion of ingredient 2, dispersing on a roller mill, heating and mixing the dispersion together with ingredient **1,** the remainder of ingredient 2, and ingredients 3 to 5.
B: Heating ingredients 6 and 7, adding them to the mixture of A, emulsifying, cooling.
C: Adding ingredient 9 to the emulsion of B to prepare a creamy lipstick.

The creamy lipstick thus obtained was lightly spreadable, was neither sticky nor greasy, and formed a long-lasting film on the lip. Since the surfactant composition within the scope of the invention was used, the preparation of the cosmetic composition was easy.

### [Example 25] Rinse-off cosmetic pack

| Ingredient | | % |
|---|---|---|
| 1. | Dimethicone 6CS | 2.7 |
| 2. | Cyclopentasiloxane | 3 |
| 3. | Surfactant composition of Example 9 | 2.3 |
| 4. | Kaolin | 30 |
| 5. | Carbomer | 0.4 |
| 6. | BG | 10 |
| 7. | Glycerin | 20 |
| 8. | Preservative | 0.1 |
| 9. | Fragrance | 0.1 |
| 10. | Water | 31.4 |
| Total | | 100.0 |

### (Preparation method)

A: Mixing ingredients 1 to 3 and 8.
B: Uniformly mixing ingredients 5 to 7 and 10, then mixing ingredients 4 and 9, and stirring.
C: Adding the mixture of A to the mixture of B and emulsifying to prepare a rinse-off cosmetic pack.

The rinse-off cosmetic pack thus obtained was lightly spreadable on application, showed an excellent rinsing effect, left a moist, non-sticky, skin-smoothing feeling after rinse-off, and showed a quite pleasant feeling on use and stability. Since the surfactant composition within the scope of the invention was used, the preparation of the cosmetic composition was easy.

### [Example 26] W/O sunscreen milky lotion

| Ingredient | | % |
|---|---|---|
| 1. | KSG-210 (*1) | 3 |
| 2. | KSG-15 (*2) | 2 |
| 3. | KF-6028 (*3) | 1 |
| 4. | Dimethicone 6CS | 5 |
| 5. | Cyclopentasiloxane | 36 |
| 6. | Isotridecyl isononanoate | 4 |
| 7. | Hydrophobized microparticulate titanium oxide | 15 |
| 8. | Hydrophobized microparticulate zinc oxide | 10 |
| 9. | Surfactant composition of Example 18 | 4 |
| 10. | Silica | 0.2 |
| 11. | DPG | 2 |
| 12. | Sodium citrate | 0.2 |
| 13. | Sodium chloride | 0.5 |
| 14. | Preservative | proper |
| 15. | Fragrance | proper |
| 16. | Water | balance |
| Total | | 100.0 |

| | | |
|---|---|---|
| (*1) Mixture of dimethicone 70-80% + (dimethicone/(PEG-10/15)) crosspolymer 20-30%, by Shin-Etsu Chemical Co., Ltd. (*2) Mixture of cyclopentasiloxane 90-96% + (dimethicone/vinyl dimethicone) crosspolymer 4-10%, by Shin-Etsu Chemical Co., Ltd. (*3) PEG-9 polydimethylsiloxyethyl dimethicone by Shin-Etsu Chemical Co., Ltd. | | |

### (Preparation method)

A: Mixing ingredients 7 to 9 with a portion of ingredient 5 and a portion of ingredient 16, and uniformly dispersing them on a bead mill.
B: Uniformly mixing ingredients 1 to 4, the remainder of ingredient 5, ingredients 6 and 10.
C: Mixing ingredients 11 to 14 and the remainder of ingredient 16.
D: With stirring, adding C to B, emulsifying, and adding A and ingredient 15 to the emulsion to prepare a W/O sunscreen milky lotion.

The sunscreen milky lotion thus obtained showed a quite pleasant feeling on use, no changes with temperature and time, and high stability. Since the surfactant composition within the scope of the invention was used, the preparation of the cosmetic composition was easy.

### [Example 27] W/O sunscreen cream

| Ingredient | | % |
|---|---|---|
| 1. | KSG-840 (*1, containing component (A)) | 3 |
| 2. | KSG-43 (*2) | 3 |
| 3. | KF-6105 (*3, component (A)) | 1.4 |
| 4. | KF-56A (*4) | 11 |
| 5. | Ethylhexyl methoxysuccinate | 7 |
| 6. | KSP-100 (*5) | 2 |
| 7. | Isostearyl alcohol (component (B)) | 0.6 |
| 8. | Xanthan gum | 0.3 |
| 9. | Dipropylene glycol | 5 |
| 10. | Glycerin | 3 |
| 11. | Methylparaben | 0.1 |
| 12. | Sodium citrate | 0.2 |
| 13. | Sodium chloride | 0.5 |
| 14. | Water | balance |
| Total | | 100.0 |

| | | |
|---|---|---|
| (*1) Mixture of squalane 65-75% + (lauryl dimethicone/polyglycerin-3) crosspolymer 25-35%, by Shin-Etsu Chemical Co., Ltd. (*2) Mixture of triethylhexanoin 65-75% + (vinyl dimethicone/lauryl dimethicone) crosspolymer 25-35%, by Shin-Etsu Chemical Co., Ltd. (*3) Lauryl polyglyceryl-3 polydimethylsiloxyethyl dimethicone by Shin-Etsu Chemical Co., Ltd. (*4) Diphenylsiloxyphenyl trimethicone by Shin-Etsu Chemical Co., Ltd. (*5) (Vinyl dimethicone/methicone silsesquioxane) crosspolymer by Shin-Etsu Chemical Co., Ltd. | | |

### (Preparation method)

A: Uniformly mixing ingredients 3 and 7 and uniformly mixing with ingredient 1.
B: Uniformly mixing A, ingredients 2 and 4 to 6.
C: Uniformly mixing ingredients 8 to 14.
D: Adding C to B and emulsifying to prepare a sunscreen cream.

The sunscreen cream thus obtained showed a quite pleasant feeling on use, no changes with temperature and time, and high stability. Since the surfactant composition within the scope of the invention was used, the preparation of the cosmetic composition was easy.

### [Example 28] W/O foundation

| Ingredient | | % |
|---|---|---|
| 1. | KSG-210 (*1) | 3.5 |
| 2. | KSG-15 (*2) | 5 |
| 3. | KF-6104 (*3, component (A)) | 2 |
| 4. | Disteardimonium hectorite | 1.2 |
| 5. | Oleic acid (component (B)) | 0.1 |
| 6. | Hexyldecanol (component (B)) | 0.1 |
| 7. | Triethylhexanoin | 4.8 |
| 8. | Dimethicone 6CS | 6.5 |
| 9. | Cyclopentasiloxane | 22.6 |
| 10. | KP-578 (*4) | 0.5 |
| 11. | KTP-09W (*5) | proper |
| 12. | KTP-09R (*5) | proper |
| 13. | KTP-09Y (*5) | proper |
| 14. | KTP-09B (*5) | proper |
| 15. | BG | 5 |
| 16. | Sodium citrate | 0.2 |
| 17. | Sodium chloride | 0.5 |
| 18. | Water | 38 |
| Total | | 100.0 |

| | | |
|---|---|---|
| (*1) Mixture of dimethicone 70-80% + (dimethicone/(PEG-10/15)) crosspolymer 20-30%, by Shin-Etsu Chemical Co., Ltd. (*2) Mixture of cyclopentasiloxane 90-96% + (dimethicone/vinyl dimethicone) crosspolymer 4-10%, by Shin-Etsu Chemical Co., Ltd. (*3) Polyglyceryl-3 polydimethylsiloxyethyl dimethicone by Shin-Etsu Chemical Co., Ltd. (*4) (Acrylate/ethylhexyl acrylate/dimethicone methacrylate) copolymer by Shin-Etsu Chemical Co., Ltd. (*5) KF-9909-treated coloring inorganic pigments, W: white, R: red, Y: yellow, B: black, by Shin-Etsu Chemical Co., Ltd. | | |

### (Preparation method)

A: Uniformly mixing ingredients 10 to 14 and a portion of ingredient 9 on a roll mill.
B: Uniformly mixing ingredients 3, 5 and 6.
C: Uniformly mixing B, ingredients 1, 2, 4, 7 to 9.
D: Uniformly mixing ingredients 15 to 18.
E: Adding D to C, emulsifying, adding A thereto, and mixing to prepare a W/O foundation.

The W/O foundation thus obtained showed a quite pleasant feeling on use, no changes with temperature and time, and high stability. Since the surfactant composition within the scope of the invention was used, the preparation of the cosmetic composition was easy.

### [Example 29] W/O stick foundation

| Ingredient | | % |
|---|---|---|
| 1. | KSG-710 (*1, containing component (A)) | 4 |
| 2. | KF-6038 (*2) | 1.5 |
| 3. | Inulin stearate (*3) | 2 |
| 4. | Ceresin | 6 |
| 5. | KF-56A (*4) | 11.5 |
| 6. | Ethylhexyl methoxysuccinate | 5 |
| 7. | KMP-590 (*5) | 1.5 |
| 8. | Isotridecyl isononanoate | 4 |
| 9. | KF-6105 (*6, component (A)) | 1 |
| 10. | Isostearic acid (component (B)) | 0.2 |
| 11. | KTP-09W (*7) | 6.5 |
| 12. | KTP-09R (*7) | proper |
| 13. | KTP-09Y (*7) | proper |
| 14. | KTP-09B (*7) | proper |
| 15. | DPG | 6 |
| 16. | Methylparaben | 0.1 |
| 17. | Sodium citrate | 0.2 |
| 18. | Sodium chloride | 0.5 |
| 19. | Water | balance |
| Total | | 100.0 |

| | | |
|---|---|---|
| (*1) Mixture of dimethicone 70-80% + (dimethicone/polyglycerin-3) crosspolymer 20-30%, by Shin-Etsu Chemical Co., Ltd. (*2) Lauryl PEG-9 polydimethylsiloxyethyl dimethicone by Shin-Etsu Chemical Co., Ltd. (*3) Rheopearl ISK2 by Ciba Flour Milling Co., Ltd. (*4) Diphenylsiloxyphenyl trimethicone by Shin-Etsu Chemical Co., Ltd. (*5) Polymethylsilsesquioxane by Shin-Etsu Chemical Co., Ltd. (*6) Lauryl polyglyceryl-3 polydimethylsiloxyethyl dimethicone by Shin-Etsu Chemical Co., Ltd. (*7) KF-9909-treated coloring inorganic pigments, W: white, R: red, Y: yellow, B: black, by Shin-Etsu Chemical Co., Ltd. | | |

### (Preparation method)

A: Uniformly mixing ingredients 9 and 10.
B: Dispersing A, ingredients 8, 11 to 14 on a roll mill.
C: Heating B and ingredients 1 to 7 at 95°C and uniformly mixing.
D: Uniformly mixing ingredients 15 to 19, and heating at 85°C.
E: Adding D to C, emulsifying, filling a stick container with the emulsion, and slowly cooling to prepare a stick foundation.

The W/O stick foundation thus obtained showed a pleasant feeling on use, good spreading, lasting quality, and formulation stability. Since the surfactant composition within the scope of the invention was used, the preparation of the cosmetic composition was easy.

### [Example 30] Polyhydric alcohol-in-oil emulsified cream

| Ingredient | | % |
|---|---|---|
| 1. | KSG-710 (*1, containing component (A)) | 5 |
| 2. | KSG-15 (*2) | 20 |
| 3. | KSG-16 (*3) | 35 |
| 4. | KF-6104 (*4, component (A)) | 1 |
| 5. | Cyclopentasiloxane | 5 |
| 6. | Hexyldecanol (component (B)) | 0.4 |
| 7. | KF-7312J (*5) | 4 |
| 8. | KSP-300 (*6) | 5 |
| 9. | Preservative | proper |
| 10. | Fragrance | proper |
| 11. | Glycerin | 5 |
| 12. | BG | 10 |
| Total | | 100.0 |

| | | |
|---|---|---|
| (*1) Mixture of dimethicone 70-80% + (dimethicone/polyglycerin-3) crosspolymer 20-30%, by Shin-Etsu Chemical Co., Ltd. (*2) Mixture of cyclopentasiloxane 90-96% + (dimethicone/vinyl dimethicone) crosspolymer 4-10%, by Shin-Etsu Chemical Co., Ltd. (*3) Mixture of dimethicone 70-80% + (dimethicone/vinyl dimethicone) crosspolymer 20-30%, by Shin-Etsu Chemical Co., Ltd. (*4) Polyglyceryl-3 polydimethylsiloxyethyl dimethicone by Shin-Etsu Chemical Co., Ltd. (*5) Solution of 50% trimethylsiloxysilicic acid in cyclopentasiloxane by Shin-Etsu Chemical Co., Ltd. (*6) (Diphenyl dimethicone/vinyl diphenyl dimethicone/silsesquioxane) crosspolymer by Shin-Etsu Chemical Co., Ltd. | | |

### (Preparation method)

A: Uniformly mixing ingredients 4 and 6 and uniformly mixing the mix with ingredient 1.
B: Adding ingredients 2, 3, 5, 7, 8 and 10 to A and uniformly mixing.
C: Mixing ingredients 9, 11 and 12.
D: Adding C to B and uniformly emulsifying to prepare a cream.

The polyhydric alcohol-in-oil emulsified cream thus obtained was lightly spreadable and neither sticky nor greasy, and showed moist feeling. Since the surfactant composition within the scope of the invention was used, the preparation of the cosmetic composition was easy.

### [Example 31] W/O cream

| Ingredient | | % |
|---|---|---|
| 1. | KSG-710 (*1, containing component (A)) | 3 |
| 2. | KSG-19 (*2) | 1 |
| 3. | KF-6104 (*3, component (A)) | 0.5 |
| 4. | Oleyl alcohol (component (B)) | 0.15 |
| 5. | Cyclopentasiloxane | 8.65 |
| 6. | KSP-101 (*4) | 2 |
| 7. | BG | 5 |
| 8. | Diglycerin | 5 |
| 9. | PEG-12 | 15 |
| 10. | Phenoxyethanol | 0.3 |
| 11. | Capsicum frutescens extract | proper |
| 12. | Glycerin | balance |
| Total | | 100.0 |

| | | |
|---|---|---|
| (*1) Mixture of dimethicone 70-80% + (dimethicone/polyglycerin-3) crosspolymer 20-30%, by Shin-Etsu Chemical Co., Ltd. (*2) Mixture of dimethicone 80-90% + (dimethicone/vinyl dimethicone) crosspolymer 10-20%, by Shin-Etsu Chemical Co., Ltd. (*3) Polyglyceryl-3 polydimethylsiloxyethyl dimethicone by Shin-Etsu Chemical Co., Ltd. (*4) (Vinyl dimethicone/methicone silsesquioxane) crosspolymer by Shin-Etsu Chemical Co., Ltd. | | |

### (Preparation method)

A: Uniformly mixing ingredients 3 and 4 and uniformly mixing the mix with ingredient 1.
B: Uniformly mixing A, ingredients 2, 5 and 6.
C: Uniformly mixing ingredients 7 to 12.
D: Adding C to B, and emulsifying to prepare a W/O cream.

The W/O cream thus obtained showed a quite pleasant feeling on use, no changes with temperature and time, and high stability. Since the surfactant composition within the scope of the invention was used, the preparation of the cosmetic composition was easy.

### [Example 32] O/W cream

| Ingredient | | % |
|---|---|---|
| 1. | KSG-15 (*1) | 8 |
| 2. | KSG-16 (*2) | 30 |
| 3. | Cyclopentasiloxane | 10 |
| 4. | BG | 3 |
| 5. | KF-6100 (*3, component (A)) | 0.6 |
| 6. | KF-6104 (*4, component (A)) | 0.3 |
| 7. | Hexyldecanol (component (B) | 0.1 |
| 8. | (Ammonium acryloyldimethyltaurate/VP) copolymer aqueous solution (*5) | 13 |
| 9. | Aqueous solution of (acrylamide/sodium acryloyldimethyltaurate) copolymer/ isohexadecane/Polysorbate 80 (*6) | 0.6 |
| 10. | 1% sodium chloride in water | 8 |
| 11. | Water | 26.5 |
| Total | | 100.0 |

| | | |
|---|---|---|
| (*1) Mixture of cyclopentasiloxane 90-96% + (dimethicone/vinyl dimethicone) crosspolymer 4-10%, by Shin-Etsu Chemical Co., Ltd. (*2) Mixture of dimethicone 70-80% + (dimethicone/vinyl dimethicone) crosspolymer 20-30%, by Shin-Etsu Chemical Co., Ltd. (*3) Polyglyceryl-3 disiloxane dimethicone by Shin-Etsu Chemical Co., Ltd. (*4) Polyglyceryl-3 polydimethylsiloxyethyl dimethicone by Shin-Etsu Chemical Co., Ltd. (*5) Aristoflex AVC by Clariant (*6) Simulgel 600 by Seppic | | |

### (Preparation method)

A: Uniformly mixing ingredients 5 to 7.
B: Uniformly mixing A and ingredients 4, 8 to 11.
C: Uniformly mixing ingredients 1 to 3.
D: Adding the mixture of C to the mixture of B, and emulsifying to prepare an O/W cream.

The O/W cream thus obtained showed a quite pleasant feeling on use, no changes with temperature and time, and high stability. Since the surfactant composition within the scope of the invention was used, the preparation of the cosmetic composition was easy.

### [Example 33] Deodorant spray

| Ingredient | | % |
|---|---|---|
| 1. | Aluminum chlorohydrate | 30 |
| 2. | Silica | 15 |
| 3. | Cyclopentasiloxane | 10 |
| 4. | Talc treated with KF-9909 (*1) | 14.88 |
| 5. | Fragrance | 0.1 |
| 6. | BHT | 0.02 |
| 7. | Zinc oxide | 5 |
| 8. | Triclosan | 0.1 |
| 9. | Isopropyl myristate | 21.6 |
| 10. | Octyl dodecanol (component (B)) | 0.3 |
| 11. | KF-6105 (*2. component (A)) | 3 |
| Total | | 100.0 |

| | | |
|---|---|---|
| (*1) Triethoxysilylethyl polydimethylsiloxyethylhexyl dimethicone by Shin-Etsu Chemical Co., Ltd. (*2) Lauryl polyglyceryl-3 polydimethylsiloxyethyl dimethicone by Shin-Etsu Chemical Co., Ltd. | | |

### (Preparation method)

A: Uniformly mixing ingredients 10 and 11.
B: Uniformly mixing ingredients 2 to 4, 6 to 9, and the mix of A.
C: Uniformly mixing ingredient 1 with the mix of B, uniformly dispersing and mixing on a mixer.
D: Adding ingredient 5 to the mixture of C and uniformly mixing.
E: Filling a spray container with 10 parts by weight of the mixture of D and 90 parts by weight of liquefied propane gas (LPG) to prepare a deodorant spray.

The deodorant spray thus obtained showed neither excessively dry feeling nor sticky feeling and its deodorant effect was lasting. Since the surfactant composition within the scope of the invention was used, the preparation of the cosmetic composition was easy.

### [Example 34] Lipstick

| Ingredient | | % |
|---|---|---|
| 1. | Candelilla wax | 4 |
| 2. | Polyethylene | 2 |
| 3. | Microcrystalline wax | 3 |
| 4. | Ceresin | 7 |
| 5. | KP-561P (*1) | 15 |
| 6. | KF-6105 (*2, component (A)) | 3 |
| 7. | Macadamia nut seed oil | 28 |
| 8. | Diisostearyl malate (component (B)) | 1 |
| 9. | Hydrogenated polyisobutene | 19 |
| 10. | Isotridecyl isononanoate | 18 |
| 11. | Pigment | proper |
| 12. | Mica | proper |
| Total | | 100.0 |

| | | |
|---|---|---|
| (*1) (Acrylate/stearyl acrylate/dimethicone methacrylate) copolymer by Shin-Etsu Chemical Co., Ltd. (*2) Lauryl polyglyceryl-3 polydimethylsiloxyethyl dimethicone by Shin-Etsu Chemical Co., Ltd. | | |

### (Preparation method)

A: Uniformly mixing ingredients 6 and 8.
B: Heating ingredients 1 to 5, 7, 9, 10 and the mixture of A at 90°C and uniformly mixing.
C: Uniformly mixing ingredients 11 and 12 with the mixture of B at 80°C, filling a stick container with the mixture, and slowly cooling to prepare a lipstick.

The lipstick thus obtained was lightly spreadable, was neither sticky nor greasy, and formed a long-lasting film on the lip. Since the surfactant composition within the scope of the invention was used, the preparation of the cosmetic composition was easy.

### [Example 35] W/O cream

| Ingredient | | % |
|---|---|---|
| 1. | KSG-710 (*1, containing component (A)) | 4 |
| 2. | KSG-15 (*2) | 1 |
| 3. | KF-6104 (*3, component (A)) | 2.5 |
| 4. | Polyglyceryl-2 triisostearate (component (B)) | 1.5 |
| 5. | Dimethicone 6CS | 12 |
| 6. | BG | 8 |
| 7. | Ethanol | 5 |
| 8. | Sodium citrate | 0.2 |
| 9. | Sodium chloride | 0.5 |
| 10. | Water | 65.3 |
| Total | | 100.0 |

| | | |
|---|---|---|
| (*1) Mixture of dimethicone 70-80% + (dimethicone/polyglycerin-3) crosspolymer 20-30%, by Shin-Etsu Chemical Co., Ltd. (*2) Mixture of cyclopentasiloxane 90-96% + (dimethicone/vinyl dimethicone) crosspolymer 4-10%, by Shin-Etsu Chemical Co., Ltd. (*3) Polyglyceryl-3 polydimethylsiloxyethyl dimethicone by Shin-Etsu Chemical Co., Ltd. | | |

### (Preparation method)

A: Uniformly mixing ingredients 3 and 4 and uniformly mixing the mix with ingredient 1.
B: Uniformly mixing the mixture of A with ingredients 2 and 5.
C: Uniformly mixing ingredients 6 to 10.
D: Adding the mixture of C to the mixture of B and emulsifying to prepare a W/O cream.

The W/O cream thus obtained showed a quite pleasant feeling on use, no changes with temperature and time, and high stability. Since the surfactant composition within the scope of the invention was used, the preparation of the cosmetic composition was easy.

### [Example 36] Pressed foundation

| Ingredient | % |
|---|---|
| 1. Ethylhexyl methoxysuccinate | 4.5 |
| 2. KF-56A (*1) | 4 |
| 3. Sorbitan sesquiisostearate (component (A)) | 0.2 |
| 4. Triethyl citrate (component (B)) | 0.05 |
| 5. KSP-100 (*2) | 2 |
| 6. KSP-300 (*3) | 3 |
| 7. Polymethylsilsesquioxane | 3.5 |
| 8. Polyethylene | 1 |
| 9. Barium sulfate | 4 |
| 10. KTP-09W (*4) | 9 |
| 11. KTP-09R, Y, B (*4) | 1 |
| 12. KF-9909-treated mica (*5) | 40 |
| 13. KF-9909-treated talc (*5) | balance |
| Total | 100.0 |

| | |
|---|---|
| (*1) Diphenylsiloxyphenyl trimethicone by Shin-Etsu Chemical Co., Ltd. (*2) (Vinyl dimethicone/methicone silsesquioxane) crosspolymer by Shin-Etsu Chemical Co., Ltd. (*3) (Diphenyl dimethicone/vinyl diphenyl dimethicone/silsesquioxane) crosspolymer by Shin-Etsu Chemical Co., Ltd. (*4) KF-9909-treated coloring inorganic pigments, W: white, R: red, Y: yellow, B: black, by Shin-Etsu Chemical Co., Ltd. (*5) Triethoxysilylethyl polydimethylsiloxy-ethylhexyl dimethicone by Shin-Etsu Chemical Co., Ltd. | |

### (Preparation method)

A: Uniformly mixing ingredients 3 and 4.
B: Uniformly mixing the mixture of A with ingredients 1 and 2.
C: Uniformly mixing ingredients 4 to 13 on a blender mill.
D: Adding the mixture of B to the mixture of C, uniformly mixing, filling a mold with the mixture and molding to prepare a pressed foundation.

The pressed foundation thus obtained showed a quite pleasant feeling on use, no changes with temperature and time, and high stability. Since the surfactant composition within the scope of the invention was used, the preparation of the cosmetic composition was easy.

## Claims

1. A surfactant composition comprising
(A) a nonionic surfactant having hydrophilic groups and at least two hydroxy groups per hydrophilic group, and
(B) an oily component having only one hydroxy group, which is liquid at 25°C, the surfactant composition having a viscosity at 25°C which is less than 50% of the viscosity of component (A).

2. The surfactant composition of claim 1 wherein the ratio of component (A) to component (B) is from 70:30 to 95:5 in weight ratio, the surfactant composition having a viscosity at 25°C of 100 to 50,000 mPa·s.

3. The surfactant composition of claim 1 wherein component (A) is a polyglycerin-modified silicone.

4. The surfactant composition of claim 3 wherein component (A) is a straight or branched polyglycerin-modified silicone.

5. The surfactant composition of claim 1 wherein component (B) is at least one compound selected from aliphatic alcohols and ester oils.

6. The surfactant composition of claim 5 wherein component (B) is a branched aliphatic alcohol of 16 to 24 carbon atoms.

7. The surfactant composition of claim 1 wherein component (B) has a solubility of less than 1 g in 100 g of purified water at 25°C.

8. A cosmetic composition comprising 0.1 to 40% by weight based on the overall cosmetic composition of the surfactant composition of any one of claims 1 to 7.

9. The cosmetic composition of claim 8 which is a water-in-oil emulsion cosmetic composition.
